(19) Europäisches Patentamt
European Patent Office
Office européen des brevets

(11) Publication number: **0 097 446**
**B1**

(12) **EUROPEAN PATENT SPECIFICATION**

(45) Date of publication of patent specification: **26.02.86**

(51) Int. Cl.[4]: **C 07 D 499/00,** C 07 F 7/10, A 61 K 31/43

(21) Application number: **83303268.3**

(22) Date of filing: **07.06.83**

(54) **Penicillin derivatives and process for preparation of the same.**

(30) Priority: **21.06.82 JP 107171/82**
**24.12.82 JP 233967/82**
**10.02.83 JP 21200/83**

(43) Date of publication of application:
**04.01.84 Bulletin 84/01**

(45) Publication of the grant of the patent:
**26.02.86 Bulletin 86/09**

(84) Designated Contracting States:
**CH DE FR GB IT LI NL**

(56) References cited:
**EP-A-0 013 067**
**US-A-4 058 521**

(73) Proprietor: **TAIHO PHARMACEUTICAL COMPANY LIMITED**
**2-9, Kanda Tsukasa-cho Chiyoda-ku**
**Tokyo 101 (JP)**

(72) Inventor: **Micetich, Ronald G.**
**12, Braeside Terrace**
**Sherwood Park Alberta T-8A-3V-6 (CA)**
Inventor: **Yamabe, Shigeru**
**1-2-7, Sumiyoshi-kamokogahara Higashinada-ku**
**Kobe-shi Kyogo-ken (JP)**
Inventor: **Yamazaki, Tomio**
**125-46, Aza-Higashinakazu Okuno Aizumi-cho**
**Itano-gun Tokushima-ken (JP)**
Inventor: **Ishida, Naobumi**
**1-163, Aza-Marusu Hiroshima Matsushige-cho**
**Itano-gun Tokushima-ken (JP)**
Inventor: **Tanaka, Motoaki**
**13-54, Matsuoka Kawauchi-cho**
**Tokushima-shi Tokushima-ken (JP)**
Inventor: **Ishizawa, Takeshi**
**41-15, Aza-Ejiriyama Mitsuishi**
**Naruto-cho Naruto-shi Tokushima-ken (JP)**
Inventor: **Kajitani, Makoto**
**831, Nishiyama Kamihachiman-cho**
**Tokushima-shi Tokushima-ken (JP)**

**EP 0 097 446 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

(74) Representative: **Huskisson, Frank Mackie et al**
**FITZPATRICKS 4 West Regent Street**
**Glasgow G2 1RS Scotland (GB)**

This invention relates to penicillin derivatives and pharmaceutically acceptable salts thereof and esters thereof, and also to a process for preparing them.

Of the commercially available antibiotics, ß-lactam type antibiotics having a ß-lactam ring, namely penicillins and cephalosporins, are best known and frequently used. Although widely used as useful chemotherapeutic drugs, the ß-lactam type antibiotics can not achieve satisfactory effects against some types of microorganisms because of resistance of the microorganism to the ß-lactam type antibiotics. The resistance thereof are usually attributable to ß-lactamase produced by the microorganism. The ß-lactamase is an enzyme which acts to cleave the ß-lactam ring of the ß-lactam type antibiotic, thereby causing the antibiotic to lose its antimicrobial activity. For this reason, the action of ß-lactamase must be eliminated or inhibited so as to enable the ß-lactam type antibiotic or produce satisfactory effects. The elimination or inhibition of the ß-lactamase activity can be achieved by ß-lactamase inhibitors, which are used conjointly with the ß-lactam type antibiotic to increase the antimicrobial activity of the antibiotic.

It is an object of the present invention to provide novel compounds having excellent ß-lactamase inhibitory action.

It is another object of the invention to provide processes for preparing the foregoing compounds.

It is a further object of the invention to provide excellent ß-lactamase inhibitory agents.

It is a still further object of the invention to provide pharmaceutical compositions which, when combined with ß-lactam type antibiotics, can increase the antibacterial activity of the antibiotics.

The foregoing objects and other features of the invention will be described below in detail.

The penicillin derivatives of the present invention are represented by the formula

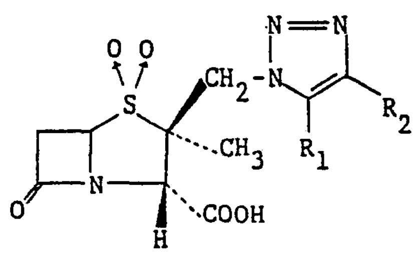

(I)

wherein $R_1$ and $R_2$, which may be the same or different, represent hydrogen atoms, tri-$C_{1-6}$-alkylsilyl groups, carboxyl groups, carboxyl groups forming a pharmaceutically acceptable salt or esterified carboxyl groups.

The penicillin derivatives of the present invention, pharmaceutically acceptable salts thereof and esters thereof are all novel compounds and have ß-lactamase inhibitory properties, hence useful as ß-lactamase inhibitory agents.

The penicillin derivatives of the invention, when used in combination with a known ß-lactam type antibiotic, can increase the antimicrobial activity of the ß-lactam type antibiotic.

Examples of antibiotics which can be used conjointly with the compounds of the present invention are ß-lactam antibiotics which exhibit antibacterial action against gram-positive or gram-negative bacteria and which include commonly used penicillins such as ampicillin, amoxicillin, hetacillin, ciclacillin, mecillinam, carbenicillin, sulbenicillin, ticarcillin, piperacillin, apalcillin, methicillin, mezlocillin and salts thereof; esters of penicillins such as bacampicillin, carindacillin, talampicillin, carfecillin and pivmecillinam, cephalosporins such as cephaloridine, cephalothin, cephapirin, cephacetrile, cefazolin, cephalexin, cefradine, cefotiam, cefamandole, cefuroxime, cefoxitin, cefmetazole, cefsulodin, cefoperazone, cefotaxime, ceftizoxime, cefmenoxime, latamoxef, cefaclor, cefroxadine, cefatrizine, cefadroxil, cephaloglycin, and salts thereof, among which it is preferred to use ampicillin, amoxicillin, piperacillin, bacampicillin, talampicillin, pivmecillinam and cephalexin. The ß-lactam antibiotic is usually used in an amount of about 0.1 to about 10 parts by weight, preferably about 0.2 to about 5 parts by weight, per part by weight of the compound of the invention.

The trialkylsilyl groups represented by $R_1$ and $R_2$ in the formula (I) of the present compounds are those having, for example, straight-chain or branched-chain alkyl groups containing 1 to 6 carbon atoms such as methyl, ethyl, propyl, butyl, isobutyl, pentyl, hexyl, etc.

The esterified carboxyl groups represented by $R_1$ and $R_2$ of the formula (I) are expressed by the formula COOR'. The groups of R' constituting the group COOR' are ester residues which are carboxyl-protecting groups acceptable in synthesis of compounds of the formula (I) and which are pharmaceutically acceptable. A pharmaceutically acceptable ester having such residue is an ester which is easily hydrolyzed *in vivo* and which is a non-poisonous ester capable of rapidly decomposing in the blood or tissue of humans, thereby producing the corresponding acid of the formula (I). Examples of the ester residues are any of the groups to be commonly used in preparing ß-lactam type antibiotics, such as those disclosed in Japanese Unexamined Patent Publication No. 81380/1974 and H. E. Flynn, "Cephalosporins and Penicillins, Chemistry and Biology" (published in 1972 by Academic Press). Specific examples of thereof are methyl, ethyl, propyl, isopropyl, butyl, tert-butyl, pentyl, hexyl, decyl, undecyl, dodecyl, tetradecyl, hexadecyl, octadecyl and like $C_{1-18}$ alkyl groups, iodomethyl, chloromethyl, 2,2-dibromoethyl, 2,2,2-trichloroethyl and

like halogenated lower alkyl groups substituted with 1 to 3 halogen atoms such as Cl, Br or I; benzyl, p-methoxybenzyl, o-nitrobenzyl, p-nitrobenzyl, diphenylmethyl and like methyl group substituted with 1 to 3 phenyl groups which may be unsubstituted or may be substituted with methoxy or nitro on the phenyl ring; methoxymethyl, ethoxymethyl, n-propyloxymethyl, iso-propyloxymethyl, n-butoxymethyl, isobutoxymethyl and like lower alkoxymethyl groups; acetoxymethyl, propionyloxymethyl, n-butyryloxymethyl, iso-butyryloxymethyl, pivalyloxymethyl, 1-acetoxyethyl, 1-pivalyloxyethyl, 1-pivalyloxypropyl, 1-propionyloxybutyl and like lower alkyl-carbonyloxy-lower alkyl; cyclopentylcarbonyloxymethyl, cyclohexylcarbonyloxymethyl and like ($C_5$—$C_7$ cycloalkyl)-carbonyloxy-lower alkyl groups; benzylcarbonyloxymethyl and like benzylcarbonyloxy-lower alkyl groups; benzoyloxymethyl, benzoyloxyethyl and like benzoyloxy-lower alkyl groups, etc. The term "lower" used in conjunction with "alkyl" or "alkoxy" is intended to indicate that each alkyl or alkoxy portion therein can contain 1 to 6 carbon atoms. The alkyl or alkoxy groupings can be straight- or branched-chain groups. Other examples of the ester residues represented by R′ are 3-phthalidyl, 4-crotonolactonyl, γ-butyrolacton-4-yl, substituted or unsubstituted phenyl, (2-oxo-1,3-dioxoden-4-yl)methyl, (5-methyl-2-oxo-1,3-dioxoden-4-yl)methyl, (5-phenyl-2-oxo-1,3-dioxoden-4-yl)methyl, tetrahydropyranyl, dimethylaminoethyl, dimethylchlorosilyl, trichlorosilyl, etc. Generally in the synthesis of penicillin compounds, ester-protecting groups are used in the art to protect penicillin carboxyl groups or other carboxyl groups. While it is difficult to determine which ester-protecting group should be used, consideration are usually given to select esters in which the protecting group per se is sufficiently stable in the reaction and which does not permit cleavage of the ß-lactam ring in removal of the ester-protecting groups. Most commonly used as such ester-protecting groups are methyl group substituted with 1 to 3 phenyl groups which may be unsubstituted or may be substituted with methoxy or nitro on the phenyl ring, lower alkyl group and halogenated lower alkyl group. Examples of the ester-protecting groups which are pharmaceutically acceptable include lower alkylcarbonyloxy-lower alkyl, ($C_5$—$C_7$ cycloalkyl)-carbonyloxy-lower alkyl, benzylcarbonyloxy-lower alkyl, benzoyloxy-lower alkyl, lactonyl, (5-sub or unsub-2-oxo-1,3-dioxodene-4-yl)methyl, phthalidyl, etc.

The carboxyl groups forming a pharmaceutically acceptable salt represented by $R_1$ and $R_2$ of the formula (I) are residues of carboxylic acid salts which are pharmaceutically acceptable. Examples of such pharmaceutically acceptable salts of the present compounds are salts of sodium, potassium, lithium or like alkali metals; salts of calcium, magnesium or like alkaline earth metals; salts of cyclohexylamine, trimethylamine diethanolamine or like organic amines; salts of alginine, lysine or like basic amino acids; ammonium salts; etc.

The compounds of the present invention can be esterified by utilizing the carboxyl group at the 3α-position, and the present invention covers such esters. Examples of the esters include any of pharmaceutically acceptable esters which are easily hydrolyzed *in vivo* and transformed into the corresponding free acid, i.e., the derivative of the formula (I). Examples of the groups consisting the ester are the same as those described above as examples of the ester residues which are represented by R′ in the formula COOR′ of the esterified carboxyl group expressed by $R_1$ and $R_2$.

Preferred examples of the derivatives of the formula (I) are those of the formulae (I—1) to (I—4)

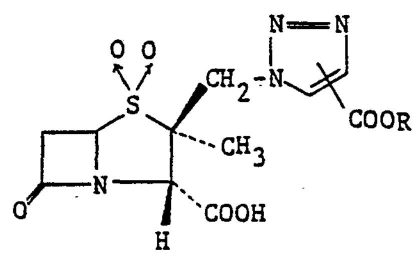

(I-1)

wherein R represents hydrogen atom or lower alkyl group.

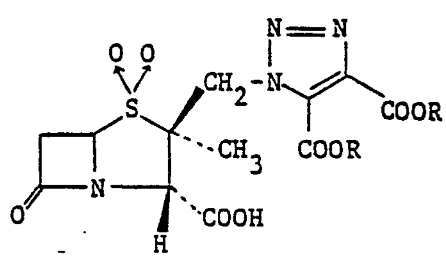

(I-2)

wherein R represents hydrogen atom or lower alkyl group.

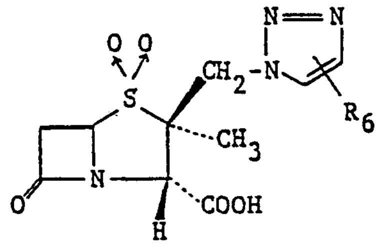

(I-3)

wherein $R_6$ represents hydrogen atom or carboxyl group.

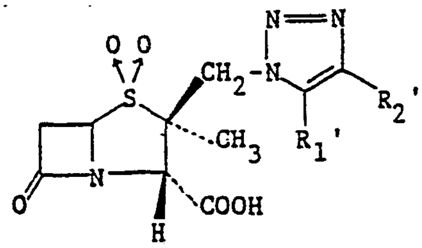

(I-4)

wherein $R_1'$ and $R_2'$ are the same as those represented by $R_1$ and $R_2$, but at least one of them is trialkylsilyl.

The penicillin derivatives of the present invention having the formula (I) can be prepared by the processes as shown in reaction equation given below according to the kinds of the groups represented by $R_1$ and $R_2$.

Reaction Equation — 1

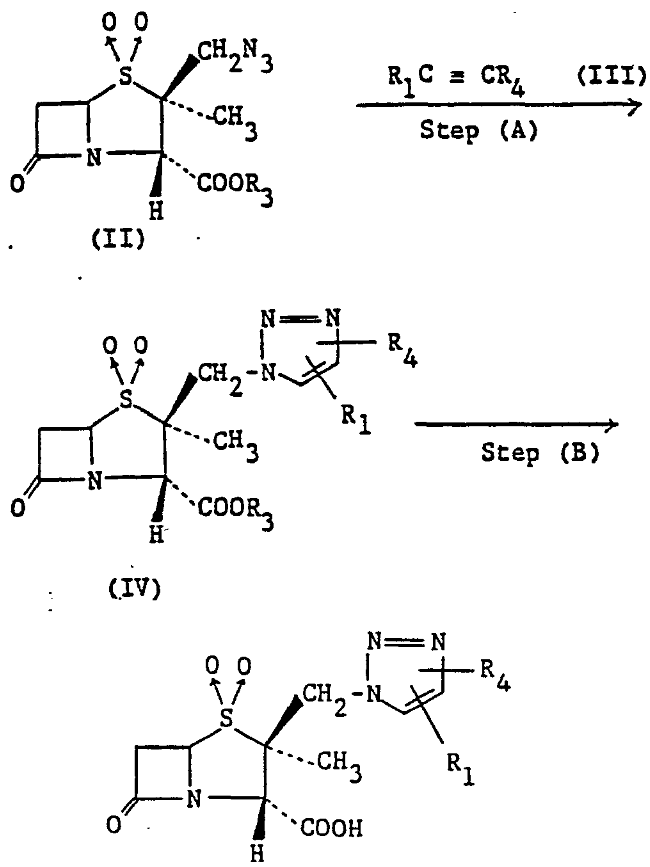


(II)

(IV)

(I—a)

wherein $R_1$ is as defined above, $R_3$ represents penicillin carboxyl-protecting group, and $R_4$ represents trialkylsilyl group, carbonyl group, carboxyl group forming a pharmaceutically acceptable salt or esterified carboxyl group.

Examples of the penicillin carboxyl-protecting group represented by $R_3$ include known groups such as any of those as described in Japanese Unexamined Patent Publication No. 81380/1974 and H. E. Flynn, "Cephalosporins and Penicillins, Chemistry and Biology" (published in 1972 by Academic Press).

Preferable examples of the groups represented by $R_3$ are methyl, ethyl, propyl, butyl, tert-butyl, trichloroethyl, benzyl, diphenyl methyl, p-nitrobenzyl, acetoxymethyl, acetoxyethyl, propionyloxyethyl, pivaloyloxyethyl, pivaloyloxypropyl, benzyloxymethyl, benzoyloxyethyl, benzylcarbonyloxymethyl, cyclohexylcarbonyloxymethyl, methoxymethyl, ethoxymethyl, benzyloxymethyl, tetrahydropyranyl, dimethylaminoethyl, dimethylchlorosilyl, trichlorosilyl, etc.

Examples of the esterified carboxyl groups and carboxyl groups forming pharmaceutically acceptable salts represented by $R_4$ are the same as those of the groups represented by $R_1$ and $R_2$ in the formula (I). Examples of the trialkylsilyl groups represented by $R_4$ are the same as those exemplified in respect of the trialkylsilyl group represented by $R_1$ and $R_2$ such as trialkylsilyl groups having methyl, ethyl, propyl, butyl, isobutyl, pentyl, hexyl, or like straight-chain or branched-chain lower alkyl groups.

The steps (A) and (B) in Reaction Equation-1 will be described below in detail.

*Step (A)*

A penicillanic acid derivative of the formula (II) is reacted with an acetylene derivative of the formula (III) to provide a compound of the formula (IV). The reaction is conducted in a suitable solvent by reacting a penicillanic acid of the formula (II) with an acetylene derivative of the formula (III) in an amount of about 1 to about 50 moles, preferably about 1 to about 10 moles, per mole of the derivative of the formula (II). Usable as the solvents are an acetylene derivative of the formula (III) as used in excess amount or toluene, xylene or like aromatic hydrocarbons, tetrahydrofuran, dioxane or like ethers, acetone or like polar solvents, etc. The reaction is performed at a temperature in the range of about 50°C to a boiling point of the solvent, or a temperature of less than 200°C in a sealed reactor, and goes to completion usually in about 2 to about 72 hours. Depending upon the kind of the penicillin carboxyl-protecting group represented by $R_3$, the compound of the formula (IV) obtained may be the preferable contemplated product of the present invention, namely an ester which is easily hydrolyzed *in vivo.* More preferably the ester thus formed is subjected to de-esterification as in the step (B) to obtain a penicillin derivative of the formula (I—a) which, in turn, is converted in the conventional manner into a pharmaceutically acceptable salt or ester thereof, when required. The compound of the formula (IV) can be made into an ester as contemplated or a pharmaceutically acceptable salt thereof by the conventional ester interchange or salt-forming reaction.

*Step (B)*

The compound of the formula (IV) is subjected to de-esterification after or without isolation from the reaction mixture resulting from the step (A), whereby a penicillin derivative of the formula (I—a) is produced.

As the de-esterification method, reduction, hydrolysis, treatment with an acid and like method can be employed for converting the carboxyl-protecting group to carboxyl group. For example, if the carboxyl-protecting group is dimethylchlorosilyl or trichlorosilyl, the reaction frequently proceeds with ease under mild hydrolysis conditions or by merely bringing the ester into contact with water. The reduction method is employed when the carboxyl-protecting group is trichloroethylbenzyl, p-nitrobenzyl, diphenyl methyl or the like. Treatment with an acid is adopted when the carboxyl-protecting group is 4-methoxybenzyl, tert-butyl, trityl, diphenylmethyl, methoxymethyl, tetrahydropyranyl or the like.

The reduction can be conducted by treating the ester of the formula (I—a) with a mixture of (a) zinc, zinc-amalgam or like metal and/or chromium chloride, chromium acetate or like chromium salt and (b) formic acid, acetic acid or like acid. Alternatively, the reduction can be conducted with use of a catalyst in hydrogen atmosphere in a solvent. Examples of the catalysts are platinum, platinum oxide, palladium, palladium oxide, palladium-barium sulfate, palladium-calcium carbonate, palladium-carbon, nickel oxide, Raney-nickel, etc. The solvents are not particularly limited so far as they do not affect the reaction, and include methanol, ethanol and like alcohols; tetrahydrofuran, dioxane and like ethers; ethyl acetate and like esters; acetic acid and like fatty acids; and a mixture of these organic solvents and water.

The acids useful for eliminating the carboxyl-protecting group of the ester of the formula (I—a) are formic acid, acetic acid and like lower fatty acids; trichloroacetic acid, trifluoroacetic acid and like trihalogenated acetic acids; hydrochloric acid, hydrofluoric acid and like hydrohalogenic acids; p-toluene-sulfonic acid, trifluoromethane-sulfonic acid and like organic sulfonic acids; and a mixture of these. In this reaction, when the acid used is in a liquid state and acts also as a solvent, it is not necessary to use other solvents. However, dimethylformamide, dichloromethane, chloroform, tetrahydrofuran, acetone and like solvents which do not affect the reaction may be used.

The penicillin derivative of the present invention having the formula (I—a) in the form of free acid can be transformed by the salt-forming reaction or esterification commonly employed in the art into a pharmaceutically acceptable salt or ester as contemplated.

If the ester residue is, for example, 3-phthalidyl, crotonolacton-4-yl, γ-butyrolactan-4-yl or like group, the penicillin derivative of the formula (I—a) can be alkylated by using 3-halogenated phthalide, 4-halogenated crotonolactone, 4-halogenated-γ-butyrolactone or the like. Suitable halogens of the foregoing halides include chlorine, bromine, iodine, etc. The reaction is carried out by dissolving the salt of the penicillin derivative of the formula (I—a) in N,N-dimethylformamide or like suitable polar organic solvent and adding an approximately equimolecular amount of a halide to the solution. The reaction temperature ranges from about 0 to about 100°C, preferably from about 15 to about 35°C. Suitable salts of the penicillin

derivative to be used in the esterification are salts of sodium, potassium or like alkali metals; salts of triethylamine, ethyldiisopropylamine, N-ethylpiperidine, N,N-dimethylaniline, N-methylmorpholine or like tertiary amines, etc. After completion of the reaction, the contemplated product can be easily separated by the conventional method and also can be purified, when required, by recrystallization, thin layer chromatography, column chromatography or like method.

The compounds of the formula (II) to be used as the starting material in the step (A) is a novel compound undisclosed in literature and can be synthesized by the method described in Japanese Patent Application No. 69142/1982 (relating to an invention accomplished by us). The disclosed method comprises the steps of reacting a metal azide with a known derivative of penicillanic acid of the formula

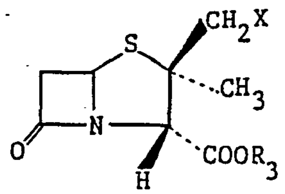

(V)

wherein X represents chlorine atom or bromine atom and $R_3$ represents penicillin carboxyl-protecting group, oxydizing the reaction mixture and subjecting the resulting compound to de-esterification.

The foregoing method will be described below in detail. The reaction between the compound of the formula (V) and the metal azide is conducted in a suitable solvent by using the metal azide in an amount of about 1 to about 50 moles, preferably about 1 to about 10 moles, per mole of the compound of the formula (V). Examples of the metal azides which can be used include those commonly used, such as sodium azide, potassium azide and like azides of alkali metals, and barium azide and like azides of alkaline earth metals. Useful solvents are not particularly limited as far as they do not affect the reaction. Examples of useful solvents are dimethylformamide, ethyl acetate, acetone, dichloromethane, tetrahydrofuran, dioxane, methanol, ethanol and like organic solvents. These organic solvents can be used singly or in mixtures. Also a mixture of such solvent and water is usable. The reaction proceeds at a temperature of usually about −20 to about 100°C, preferably about 0 to about 100°C. The resulting product can be used in subsequent oxidation without isolation, or alternatively after isolation and purification by a conventional method. The oxidation subsequent to the azide-forming reaction is conducted by using an oxidizing agent commonly employed such as permanganic acid, periodic acid, peracetic acid, performic acid, trifluoroperacetic acid, perbenzoic acid, m-chloroperbenzoic acid, hydrogen peroxide, etc. The oxidizing agent can be used in large excess, and may be employed preferably in an amount of about 1 to about 2 moles per mole of the starting compound. The oxidation is carried out usually in a suitable solvent. Useful solvents include any of those which do not affect the oxidation reaction such as chloroform, pyridine, tetrahydrofuran, dioxane, methylene chloride, carbon tetrachloride, acetic acid, formic acid, dimethylformamide, water, etc. The oxidation is performed at a temperature which is not particularly limited but generally ranges from room temperature to cooling temperature, preferably about 0 to about 30°C.

The compound thus obtained is subjected to de-esterification whereby the compound of the formula (II) can be produced. The de-esterification is effected under the same conditions as mentioned above in respect of the step (B). The process for preparing the compound of the formula (II) is described in detail in reference examples to be set forth later.

Reaction Equation — 2

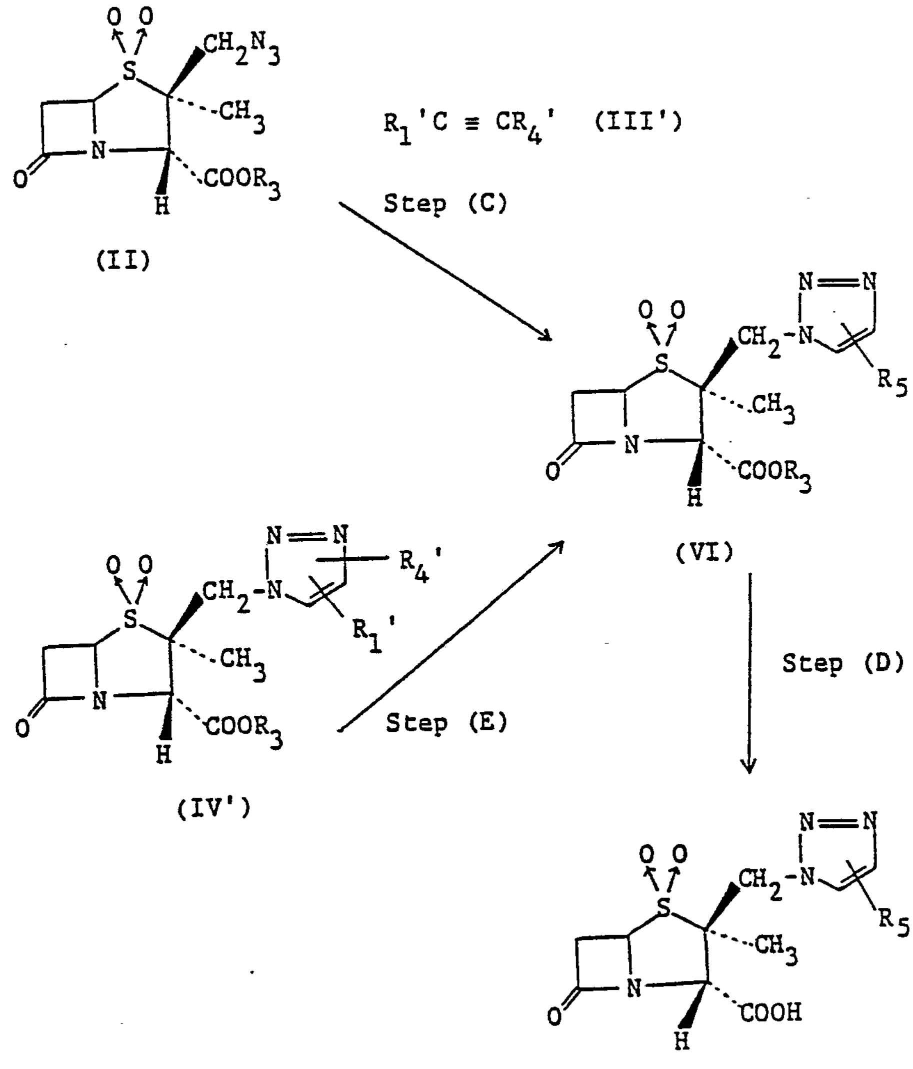


(II) (VI) (IV') (I-b)

In the foregoing formulae, $R_3$ is defined above, $R_1'$ and $R_4'$ are the same groups as those represented by $R_1$ and $R_4$ and at least one of them is trialkylsilyl group, and $R_5$ represents hydrogen atom, carboxyl group, carboxyl group forming a pharmaceutically acceptable salt or esterified carboxyl group.

Examples of the esterified carboxyl group and carboxyl group forming a pharmaceutically acceptable salt represented by $R_5$ in the foregoing formulae are the same as those mentioned above as groups represented by $R_1$ and $R_2$ in the formula (I).

The compound of the formula (I) wherein at least one of $R_1$ and $R_2$ is hydrogen atom, namely the compound of the formula (I—b), can be prepared by the process shown above in Reaction Equation-2. The steps in the process are set forth below in detail.

*Step (C)*

The compound of the formula (II) is reacted with a compound of the formula (III') in a solvent such as dichloromethane, dichloroethane, chloroform or like halogenated hydrocarbons. During this reaction, reaction for removing the trialkylsilyl group proceeds at the same time, whereby a compound of the formula (VI) is produced. Useful solvents are not particularly limited as far as they are halogenated hydrocarbons. The reaction conditions including the reaction temperature, the proportions of the reagents to be used and the reaction time are similar to those in the step (A).

Depending upon the kind of the penicillin carboxyl-protecting group represented by $R_3$, the compound of the formula (VI) thus obtained may be the preferable product as contemplated, i.e., an ester of the penicillin derivative of the formula (I) which is easily hydrolyzed *in vivo*. More preferably the ester is subjected to de-esterification as in the step (B) so that the compound is transformed to a penicillin derivative of the present invention having the formula (I—b) which is converted, when required, in the conventional manner into a pharmaceutically acceptable salt thereof or ester thereof as contemplated.

*Step (D)*

The compound of the formula (VI) is subjected to de-esterification after or without isolation from the reaction product obtained in the step (C), whereby a penicillin derivative of the formula (I—b) is produced. The de-esterification is carried out under the same conditions as those described above in respect of the step (B).

The compound of the formula (VI) can be prepared by the process in the step (C) and also by the process to be set forth below in a step (E).

*Step (E)*

The compound of the formula (IV) obtained in the step (A) as shown in Reaction Equation-1 wherein at least one of $R_1$ and $R_4$ is trialkylsilyl, namely the compound of the formula (IV'), is subjected to reaction for removing the trialkylsilyl in the presence of potassium fluoride after or without isolation from the reaction product obtained in the step (A), whereby a compound of the formula (VI) is produced. The trialkylsilyl-removing reaction is conducted in a suitable solvent by using potassium chloride in an amount of over about 1 mole, preferably about 1 mole, and a catalyst in an amount of about 1/50 to about 1/10 mole, both per mole of the compound of the formula (IV). Useful as the catalyst is a phase transfer catalyst such as quaternary ammonium salt of crown ether or the like. Examples of useful solvents are any suitable solvents which do not affect the reaction and which include benzene, toluene, xylene or like aromatic hydrocarbons; acetonitrile, N,N-dimethylformamide, dimethylsulfoxide or like non-protonic polar solvents; etc. The reaction temperature and reaction time are appropriately determined. Generally the reaction is performed at a temperature in the range of room temperature to about 100°C, and completes in about 1 to about 10 hours.

Reaction Equation-3

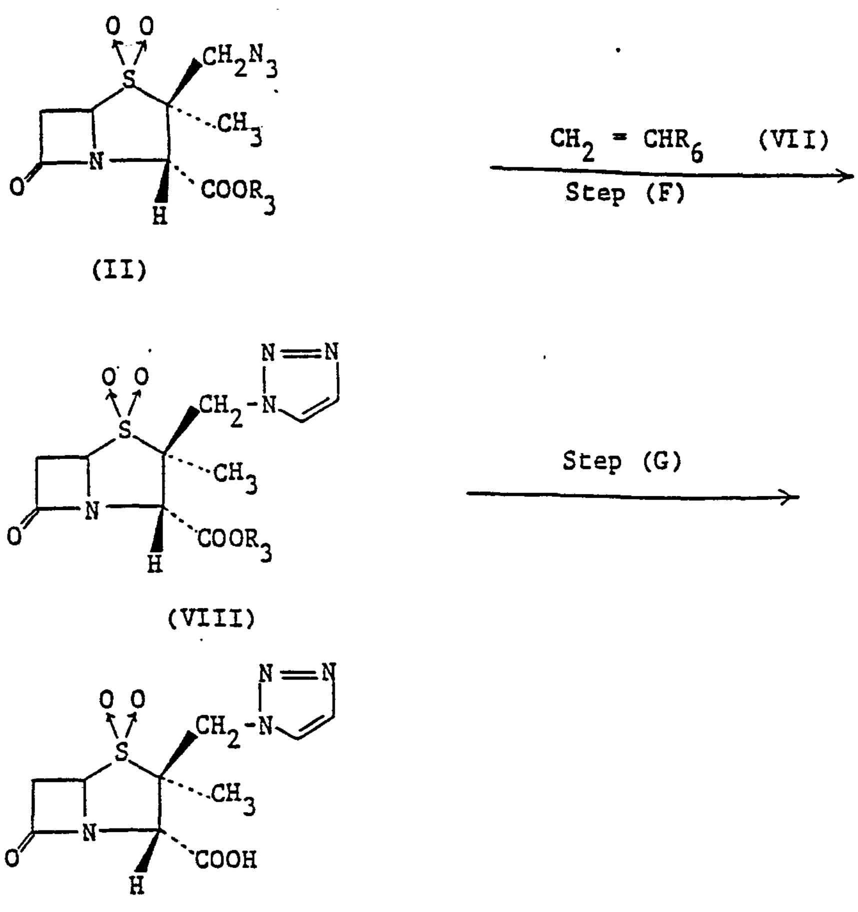


(II)

(VIII)

(I-c)

In the foregoing formulae, $R_3$ is as defined above, and $R_6$ represents acyloxy group.

Examples of the acyloxy groups represented by $R_6$ are lower acyloxy groups having 2 to 5 carbon atoms such as acetoxy, propionyloxy, butyryloxy, valeryloxy or like aliphatic acyloxy groups and benzoyloxy or like aromatic acyloxy groups, etc.

The compound of the formula (I) wherein $R_1$ and $R_2$ are hydrogen atoms, namely the compound of the formula (I—c), can be produced by the process as shown above in Reaction Equation-3.

The steps (F) and (G) in Reaction Equation-3 will be described below in detail.

*Step (F)*

The penicillanic acid derivative of the formula (II) is reacted with the vinyl derivative of the formula (VII) while reaction for removing the acyloxy group represented by $R_6$ in the formula (VII) is carried out, whereby a compound of the formula (VIII) is prepared. The reaction between the penicillanic acid derivative of the formula (II) and the vinyl derivative of the formula (VII) is conducted in the presence of or in the absence of a suitable solvent by using the vinyl derivative of the formula (VII) in an amount of at least about 1 mole, preferably about 1 to about 200 moles, per mole of the derivative of the formula (II), whereby there occurs simultaneously the acyloxy-removing reaction. The solvents which can be used are not particularly limited as far as they do not affect the reaction. Specific examples thereof are benzene, toluene, xylene or like aromatic hydrocarbons, tetrahydrofuran, dioxane or like ethers, etc. The reaction is effected at a temperature ranging from about 50°C to a boiling point of the solvent, or a temperature of less than 200°C in a sealed reactor, and is completed in about 2 to about 72 hours. Depending on the kind of the penicillin carboxyl-protecting group represented by $R_3$ in the formula (VIII), the compound of the formula (VIII) thus obtained may be the preferable product as contemplated, namely the ester of the penicillin derivative of the formula (I) which is easily hydrolyzed *in vivo*. More preferably the compound of the formula (VIII) thus prepared is subjected to de-esterification as in the step (B) so that the compound is converted by the conventional method into a penicillin derivative of the formula (I—c) which, in turn, is transformed by the conventional method into a pharmaceutically acceptable salt thereof or ester thereof as contemplated. The compound of the formula (VIII) can be made into a pharmaceutically acceptable salt thereof or ester thereof as contemplated by conducting an ester interchange or salt-forming reaction in the conventional manner.

*Step (G)*

The compound of the formula (VIII) is subjected to de-esterification after or without isolation from the reaction product obtained in the step (F), whereby a penicillin derivative of the formula (I-c) as contemplated is produced. The reaction conditions for de-esterification are the same as those described in the step (B).

After completion of the reaction in each step, the contemplated compound producible in each step, a pharmaceutically acceptable salt thereof and ester thereof as contemplated which is easily hydrolyzed *in vivo* can be isolated from the reaction product or, when required, can be purified by the conventional method such as recrystallization method, thin-layer chromatography, column chromatography or the like.

The penicillin derivative of the present invention, or a pharmaceutically acceptable salt thereof or ester thereof is mixed with the β-lactam type antibiotic substance to form a preparation which is orally or parenterally administered. Alternatively, the present compound and a suitable antibiotic can be separately administered. Thus the derivatives of the formula (I) can be used for treating infectious disease of human beings and other animals.

The composition of the present invention may be made into tablets, pills, capsules, granules, powders. syrups, lozenges, solutions, suspensions, etc. for oral administration and aqueous, suspending or water soluble preparations for intravenous, subcutaneous or intramuscular injections.

Carriers useful in formulating the preparations are commonly used pharmaceutically acceptable non toxic carriers such as gelatin, lactose, starch, magnesium stearate, talc, vegetable oil, animal oil polyalkylene glycol, etc. The carrier may be used with other additives such as diluents, binders, buffer agents, preservatives, glazes, disintegrators, coating agents, etc.

The daily dose of the preparation can be appropriately determined and is not particularly limited Preferably the daily dose is such that the total amount of the present compound and β-lactam antibiotic is about 1 to about 200 mg/Kg body weight for oral administration and about 1 to about 100 mg/Kg body weight for parenteral administration.

The present invention will be described below in more detail with reference to examples given below

Reference Example 1

Preparation of benzhydryl 2β-azidomethyl-2α-methylpenam-3α-carboxylate

A solution of 5.00 g of sodium azide in 53 ml of water was added to a solution of benzhydryl 2β-chloromethyl-2α-methylpenam-3α-carboxylate (5.13 g) in dimethyl formamide (155 ml). The mixture was stirred at room temperature for 4 hours. The resulting reaction mixture was poured into cooled water and the mixture was extracted with ethyl acetate. The ethyl acetate layer was washed with water, dried over magnesium sulfate and concentrated to provide 4.87 g of the contemplated product as oil in 93% yield

Infrared absorption spectrum (nujol) ν max ($cm^{-1}$):

2120, 1812, 1765

Nuclear magnetic resonance spectrum ($CDCl_3$) δ (ppm):

1.30 (3H, s), 3.25 (2H, m), 3.42 (1H, d), 3.63 (1H, d), 4.75 (1H, s), 4.76 (1H, m), 7.00 (1H, s), 7.40 (10H, s)

Reference Example 2

Preparation of benzhydryl 2β-azidomethyl-2α-methylpenam-3α-carboxylate-1,1-dioxide

To a solution of benzhydryl 2β-azidomethyl-2α-methylpenam-3α-carboxylate (7.03 g) in a mixture of acetic acid (240 ml) and water (40 ml) was added potassium permanganante (6.02 g) over a period of more than 1 hour. The mixture was stirred at room temperature for 2.5 hours. The resulting reaction mixture was diluted with ice water. The precipitate was collected by filtration, and washed with water. The resulting product was dissolved in ethyl acetate and the solution was washed with an aqueous solution of sodium hydrogencarbonate and dried over magnesium sulfate. Concentration gave 5.48 g of the contemplated product in 72% yield.

Infrared absorption spectrum (nujol) ν max ($cm^{-1}$):

2120, 1812, 1765

Nuclear magnetic resonance spectrum ($CDCl_3$) δ (ppm):

1.18 (3H, s), 3.50 (2H, d), 3.72 (1H, d), 3.93 (1H, d), 4.60 (1H, m), 4.65 (1H, s), 7.00 (1H, s), 7.36 (10H, s)

Reference Example 3

Preparation of p-nitrobenzyl 2β-azidomethyl-2α-methylpenam-3α-carboxylate

The procedure of Reference Example 1 was repeated with the exception of using as the staring material p-nitrobenzyl 2β-chloromethyl-2α-methylpenam-3α-carboxylate, affording the above contemplated compound.

Infrared absorption spectrum (KBr) ν max ($cm^{-1}$):

2120, 1798, 1760

Nuclear magnetic resonance spectrum ($CDCl_3$) δ (ppm):

1.40 (3H, s), 3.12 (1H, dd), 3.50 (2H, s), 3.62 (1H, dd), 4.83 (1H, s), 5.29 (2H, s), 5.36 (1H, dd), 7.56 (2H, d), 8.26 (2H, d)

Reference Example 4

Preparation of p-nitrobenzyl 2β-azidomethyl-2α-methylpenam-3α-carboxylate-1,1-dioxide

The procedure of Reference Example 2 was followed with the exception of using as the starting material p-nitrobenzyl 2β-azidomethyl-2α-methylpenam-3α-carboxylate, giving the above contemplated compound.

Infrared absorption spectrum (KBr) ν max ($cm^{-1}$):

2120, 1770

Nuclear magnetic resonance spectrum ($CDCl_3$) δ (ppm):

1.42 (3H, s), 3.45—3.60 (2H, m), 3.75 (1H, d), 3.96 (1H, d), 4.56—4.75 (1H, m), 4.64 (1H, s), 5.33 (2H, s), 7.56 (2H, d), 8.26 (2H, d)

Example 1

Preparation of p-nitrobenzyl 2β-(4-ethoxycarbonyl-1,2,3-triazole-1-yl)methyl-2α-methylpenam-3α-carboxylate-1,1-dioxide (Compound 1) and p-nitrobenzyl 2β-(5-ethoxycarbonyl-1,2,3-triazole-1-yl)methyl-2α-methylpenam-3α-carboxylate-1,1-dioxide (Compound 2)

A 2.1 g quantity of 2β-nitromethyl-2α-methylpenam-3α-carboxylate-1,1-dioxide and 0.63 g of ethylpropiolic acid in 62 ml of benzene was refluxed with stirring under nitrogen atmosphere for 37 hours. The solvent was removed by distillation and the residue was subjected to column chromatography on silica gel to produce as a first eluted product 0.7 g of p-nitrobenzyl 2β-(5-ethoxycarbonyl-1,2,3-triazole-1-yl)methyl-2α-methylpenam-3α-carboxylate-1,1-dioxide in amorphous form (Compound 2) in 27% yield.

Infrared absorption spectrum (KBr) ν max ($cm^{-1}$):

1795, 1755, 1727

Nuclear magnetic resonance spectrum ($CDCl_3$) δ (ppm):

1.39 (3H, s), 1.39 (3H, t), 3.48—3.60 (2H, m), 4.39 (2H, q), 4.58—4.70 (1H, m), 5.11 (1H, s), 5.14 (1H, d), 5.25 (1H, d), 5.31 (1H, d), 5.56 (1H, d), 7.54 (2H, d), 8.09 (1H, s), 8.25 (2H, d)

There was obtained as a second eluted product 1.6 g of p-nitrobenzyl 2β-(5-ethoxycarbonyl-1,2,3-triazole-1-yl)methyl-2α-methylpenam-3α-carboxylate-1,1-dioxide in amorphous form (Compound 1) in 62% yield.

Infrared absorption spectrum (KBr) ν max ($cm^{-1}$):

1800, 1760 (sh), 1733

Nuclear magnetic resonance spectrum ($CDCl_3$) δ (ppm):

1.34 (3H, s), 1.41 (3H, t), 3.50—3.65 (2H, m), 4.42 (2H, q), 4.60—4.75 (2H, m), 5.09 (2H, s), 5.36 (2H, s), 7.59 (2H, d), 8.28 (2H, d), 8.30 (1H, s)

Example 2

Preparation of p-nitrobenzyl 2β-(4-methoxycarbonyl-1,2,3-triazole-1-yl)methyl-2α-methylpenam-3α-carboxylate-1,1-dioxide (Compound 3) and p-nitrobenzyl 2β-(5-methoxycarbonyl-1,2,3-triazole-1-yl)methyl-2α-methylpenam-3α-carboxylate-1,1-dioxide (Compound 4)

The contemplated product was synthesized in the same manner as in Example 1 and eluted by column

chromatography on silica gel. There was obtained as a first eluted product p-nitrobenzyl 2β-(5-methoxycarbonyl-1,2,3-triazole-1-yl)methyl-2α-methylpenam-3α-carboxylate-1,1-dioxide in amorphous form (Compound 4) in 26% yield.

Infrared absorption spectrum (KBr) ν max ($cm^{-1}$):

1795, 1727

Nuclear magnetic resonance spectrum ($CDCl_3$) δ (ppm):

1.39 (3H, s), 3.45—3.60 (2H, m), 3.94 (3H, s), 4.58—4.70 (1H, m), 5.09 (1H, s), 5.10—5.64 (4H, m), 7.54 (2H, d), 8.10 (1H, s), 8.25 (2H, d).

There was obtained as a second eluted product p-nitrobenzyl 2β-(4-methoxycarbonyl-1,2,3-triazole-1-yl)methyl-2α-methylpenam-3α-carboxylate-1,1-dioxide in amorphous form (Compound 3) in 61% yield.

Infrared absorption spectrum (KBr) ν max ($cm^{-1}$):

1798, 1730

Nuclear magnetic resonance spectrum ($CDCl_3$) δ (ppm):

1.33 (3H, s), 3.48—3.68 (2H, m), 3.96 (3H, s), 4.56—4.76 (2H, m), 5.09 (2H, s), 5.36 (2H, s), 7.60 (2H, d), 8.28 (2H, d), 8.30 (1H, s).

## Example 3

Preparation of benzhydryl 2β-(4-methoxycarbonyl-1,2,3-triazole-1-yl)methyl-2α-methylpenam-3α-carboxylate-1,1-dioxide (Compound 5) and benzhydryl 2β-(5-methoxycarbonyl-1,2,3-triazole-1-yl)methyl-2α-methylpenam-3α-carboxylate-1,1-dioxide (Compound 6)

The contemplated product was synthesized in the same manner as in Example 1 and eluted by column chromatography on silica gel. First there was eluted benzhydryl 2β-(5-methoxycarbonyl-1,2,3-triazole-1-yl)-methyl-2α-methylpenam-3α-carboxylate-1,1-dioxide (Compound 6) in 18% yield.

Infrared absorption spectrum (KBr) ν max ($cm^{-1}$):

1800, 1727

Nuclear magnetic resonance spectrum ($CDCl_3$) δ (ppm):

1.20 (3H, s), 3.44—3.58 (2H, m), 3.91 (3H, s), 4.50—4.65 (1H, m), 5.24 (1H, d), 5.25 (1H, s), 5.45 (1H, d), 6.91 (1H, s), 7.20—7.40 (10H, m), 8.08 (1H, s).

Secondly there was eluted benzhydryl 2β-(4-methoxycarbonyl-1,2,3-triazole-1-yl)methyl-2α-methylpenam-3α-carboxylate-1,1-dioxide (compound 5) in 60% yield.

Infrared absorption spectrum (KBr) ν max ($cm^{-1}$):

1803, 1727

Nuclear magnetic resonance spectrum ($CDCl_3$) δ (ppm):

1.05 (3H, s), 3.48—3.62 (2H, m), 3.95 (3H, s), 4.55—4.75 (2H, m), 5.11 (2H, bs), 7.02 (1H, s), 7.20—7.50 (10H, m), 8.25 (1H, s).

## Example 4

Preparation of sodium 2β-(4-ethoxycarbonyl-1,2,3-triazole-1-yl)methyl-2α-methylpenam-3α-caboxylate-1,1-dioxide (Compound 7)

Hydrogenation was conducted at a low pressure and at room temperature by using 15 ml of ethyl acetate, 15 ml of water, 340 mg of p-nitrobenzyl 2β-(4-ethoxycarbonyl-1,2,3-triazole-1-yl)methyl-2α-methylpenam-3α-carboxylate-1,1-dioxide, 60 mg of 10% palladium charcoal and 110 mg of sodium hydrogencarbonate. After completion of absorption of hydrogen, the reaction mixture was filtered to separate the aqueous layer which was washed with benzene. The aqueous solution was concentrated at reduced pressure and the concentrate was subjected to column chromatography using an MCI gel, CHP—20 P (product of Mitsubishi Kasei Co., Ltd., Japan) to conduct gradient elution with a water-10% acetone water mixture. The eluate thus obtained was freeze-dried to afford 200 mg of the contemplated product (Compound 7) as white powder in 76% yield. The white powder decomposed at a temperature of more than 180°C.

Infrared absorption spectrum (KBr) ν max ($cm^{-1}$):

1782, 1720

Nuclear magnetic resonance spectrum ($D_2O$) δ (ppm):

1.39 (3H, t), 1.46 (3H, s), 3.45 (1H, dd), 3.72 (1H, dd), 4.44 (2H, q), 4.50 (1H, s), 4.96— 5.10 (1H, m), 5.18 (1H, d), 5.42 (1H, d), 8.72 (1H, s).

## Example 5

Preparation of 2β-(4-ethoxycarbonyl-1,2,3-triazole-1-yl)methyl-2α-methylpenam-3α-carboxylic acid-1,1-dioxide (Compound 8)

Hydrogenation was conducted at room temperature and at a pressure of 3 atm. by using 4.2 g of p-nitro-benzyl 2β-(4-ethoxycarbonyl-1,2,3-triazole-1-yl)methyl-2α-methylpenam-3α-carboxylate-1,1-dioxide, 1.4 g of sodium hydrogencarbonate, 800 mg of 10% palladium charcoal, 100 ml of ethyl acetate and 100 ml of water. After completion of absorption of hydrogen, the reaction mixture was filtered and the aqueous layer was separated and washed with benzene. The pH of the aqueous layer was adjusted to 1 to 2 with hydrochloric acid. The aqueous layer was extracted with ethyl acetate and the extract was dried over

magnesium sulfate. The solvent was distilled off and 3.0 g of the contemplated compound was produced in amorphous form in 97% yield.

Infrared absorption spectrum (KBr) ν max ($cm^{-1}$):

1798, 1726

Nuclear magnetic resonance spectrum (DMSO-$d_6$) δ (ppm):

1.31 (3H, t), 1.42 (3H, s), 3.31 (1H, dd), 3.73 (1H, dd), 4.32 (2H, q), 4.75—5.38 (4H, m), 8.76 (1H, s).

Example 6

Preparation of chloromethyl 2β-(4-ethoxycarbonyl-1,2,3-triazole-1-yl)methyl-2α-methylpenam-3α-carboxylate-1,1-dioxide (Compound 9)

A 2.2 g quantity of sodium hydrogencarbonate and 0.2 g of tetrabutylammonium hydrogensulfate were added with stirring at a temperature of less than 10°C to 2.4 g of 2β-(4-ethoxycarbonyl-1,2,3-triazole-1-yl)methyl-2α-methylpenam-3α-carboxylic acid-1,1-dioxide, 13.5 ml of dichloromethane and 13.5 ml of water. To the mixture was dropwise added at the same temperature 1.25 g of chloromethyl chlorosulfonate and the resulting mixture was stirred at room temperature for 30 minutes. The organic layer was separated, washed once with water and dried over magnesium sulfate. The solvent was removed by distillation and the residue was purified by column chromatography on silica gel, giving 2.2 g of the contemplated compound in amorphous form in 81% yield.

Infrared absorption spectrum (KBr) ν max ($cm^{-1}$):

1798, 1723

Nuclear magnetic resonance spectrum ($CDCl_3$) δ (ppm):

1.42 (3H, t), 1.48 (3H, s), 3.52—3.65 (2H, m), 4.36 (2H, q), 4 .60—4.78 (2H, m), 5.10 (2H, s), 5.73 (1H, d), 5.90 (1H, d), 8.31 (1H, s).

Example 7

Preparation of iodomethyl 2β-(4-ethoxycarbonyl-1,2,3-triazole-1-yl)methyl-2α-methylpenam-3α-carboxylate-1,1-dioxide (Compound 10)

A 1.73 g quantity of chloromethyl 2β-(4-ethoxycarbonyl-1,2,3-triazole-1-yl)methyl-2α-methylpenam-3α-carboxylic acid-1,1-dioxide and 1.3 g of sodium iodide were stirred in 3.4 ml of acetone at room temperature for 18 hours. To the reaction mixture was added 2.9 ml of water and the pH of the resulting mixture was adjusted to 7 to 8 with an aqueous solution of sodium hydrogencarbonate. After addition of 2.9 ml of water, the mixture was decolorized with an aqueous solution of 0.5 M sodium thiosulfate, extracted with dichloromethane, washed with water and dried over magnesium sulfate. The solvent was removed by distillation and 1.9 g of the contemplated compound was prepared in amorphous form in 90% yield.

Infrared absorption spectrum (KBr) ν max ($cm^{-1}$):

1798, 1725

Nuclear magnetic resonance spectrum ($CDCl_3$) δ (ppm):

1.42 (3H, t), 1.49 (3H, s), 3.52—3.68 (2H, m), 4.43 (2H, q), 4.59—4.78 (2H, m), 5.09 (2H, s), 5.96 (1H, d), 6.07 (1H, d), 8.32 (1H, s).

Example 8

Preparation of sodium 2β-(5-ethoxycarbonyl-1,2,3-triazole-1-yl)methyl-2α-methylpenam-3α-carboxylate-1,1-dioxide (Compound 11)

A 222 mg of the contemplated compound was prepared in the form of white powder in the same manner as in Example 4 from 0.34 p-nitrobenzyl 2β-(5-ethoxycarbonyl-1,2,3-triazole-1-yl)methyl-2α-methylpenam-3α-carboxylate-1,1-dioxide in 83% yield.

The white powder thus obtained decomposed at a temperature of over 180°C.

Infrared absorption spectrum (KBr) ν max ($cm^{-1}$):

1788, 1736

Nuclear magnetic resonance spectrum ($D_2O$) δ (ppm):

1.39 (3H, t), 1.43 (3H, s), 3.40 (1H, dd), 3.71 (1H, dd), 4.46 (2H, q), 4.57 (1H, s), 4.96—5.05 (1H, m), 5.40 (1H, d), 5.82 (1H, d), 8.34 (1H, s).

Example 9

Preparation of sodium 2β-(4-methoxycarbonyl-1,2,3-triazole-1-yl)methyl-2α-methylpenam-3α-carboxylate-1,1-dioxide (Compound 12)

A 0.18 g quantity of the contemplated product was prepared as white powder in the same manner as in Example 4 from 0.3 g of p-nitrobenzyl 2β-(4-methoxycarbonyl-1,2,3-triazole-1-yl)methyl-2α-methylpenam-3α-carboxylate-1,1-dioxide in 78% yield.

The white powder thus obtained decomposed at a temperature of over 184°C.

Infrared absorption spectrum (KBr) ν max ($cm^{-1}$):

1782, 1730

Nuclear magnetic resonance spectrum ($D_2O$) δ (ppm):

1.46 (3H, s), 3.45 (1H, dd), 3.73 (1H, dd), 3.97 (3H, s), 4.50 (1H, s), 4.81 (2H, s), 4.98—5.10 (1H, m), 5.18 (1H, d), 5.42 (1H, d), 8.72 (1H, s).

Example 10

Preparation of sodium 2β-(5-methoxycarbonyl-1,2,3-triazole-1-yl)methyl-2α-methylpenam-3α-carboxylate-1,1-dioxide (Compound 13)

A 0.19 g quantity of the contemplated compound was prepared as white powder in the same manner as in Example 4 from 0.3 g of p-nitrobenzyl 2β-(5-methoxycarbonyl-1,2,3-triazole-1-yl)methyl-2α-methylpenam-3α-carboxylate-1,1-dioxide in 82% yield.

The white powder thus obtained decomposed at a temperature of over 180°C.

Infrared absorption spectrum (KBr) ν max ($cm^{-1}$):
1778, 1730

Nuclear magnetic resonance spectrum ($D_2O$) δ (ppm):
1.41 (3H s), 3.41 (1H, dd), 3.71 (1H, dd), 3.98 (3H, s), 4.56 (1H, s), 4.95—5.08 (1H, m), 5.40 (1H, d), 5.83 (1H, d), 8.34 (1H, s).

Example 11

Preparation of p-nitrobenzyl 2α-methyl-2β-[4-(p-nitrobenzyloxycarbonyl-1,2,3-triazole-1-yl]methylpenam-3α-carboxylate-1,1-dioxide (Compound 14) and p-nitrobenzyl 2α-methyl-2β-[5-p-nitrobenzyloxycarbonyl)-1,2,3-triazole-1-yl]methylpenam-3α-carboxylate-1,1-dioxide (Compound 15)

A 4 g quantity of p-nitrobenzyl 2β-adidomethyl-2α-methylpenam-3α-carboxylate-1,1-dioxide and 8.2 g of p-nitrobenzyl acetylene carboxylate in 100 ml of benzene were refluxed under nitrogen atmosphere for 12 hours. The solvent was distilled off at reduced pressure. The residue was subjected to column chromatography on silica gel to provide 3.6 g of p-nitrobenzyl 2α-methyl-2β-[4-(p-nitrobenzyloxycarbonyl)-1,2,3-triazole-1-yl]methylpenam-3α-carboxylate-1,1-dioxide (Compound 14) and 0.9 g of p-nitrobenzyl 2α-methyl-2β-[5-p-nitrobenzyloxycarbonyl)-1,2,3-triazole-1-yl]methylpenam-3α-carboxylate-1,1-dioxide (Compound 15) both in amorphous form.

Compound 14

Infrared absorption spectrum (KBr) ν max ($cm^{-1}$):
1800, 1740

Nuclear magnetic resonance spectrum ($CDCl_3$) δ (ppm):
1.34 (3H, s), 3.3—3.8 (2H, m), 4.67 (1H, s), 4.60—4.76 (1H, m), 5.12 (2H, s), 5.37 (2H, s), 5.48 (2H, s), 7.5—7.7 (4H, m), 8.1—8.3 (4H, m), 8.37 (1H, s).

Compound 15

Infrared absorption spectrum (KBr) ν max ($cm^{-1}$):
1800, 1740

Nuclear magnetic resonance spectrum ($CDCl_3$) δ (ppm):
1.41 (3H, s), 3.3—3.7 (2H, m), 4.6—4.7 (1H, m), 5.07 (1H, s), 5.1—5.6 (4H, m), 5.46 (2H, s), 7.4—7.7 (4H, m), 8.15 (1H, s), 8.1—8.4 (4H, m).

Example 12

Preparation of dipotassium 2β-(4-carboxy-1,2,3-triazole-1-yl)methyl-2α-methylpenam-3α-carboxylate-1,1-dioxide (Compound 16)

Hydrogenation was conducted in 100 ml of ethyl acetate and 100 ml of water at room temperature for 1 hour by using 3.6 g of p-nitrobenzyl 2α-methyl-2β-[4-nitrobenzyloxycarbonyl-1,2,3-triazole-1-yl]-methylpenam-3α-carboxylate-1,1-dioxide, 2.0 g sodium hydrogencarbonate and 0.68 g of 10% palladium charcoal, catalyst. Thereafter the aqueous layer was separated and was washed once with ethyl acetate, and the pH thereof was adjusted to 1.5 to 1.7 with 6 N hydrochloric acid. The aqueous solution was saturated with sodium chloride and extracted a few times with ethyl acetate. The ethyl acetate solutions thus formed were collected and dried over magnesium sulfate. The solvent was distilled off at reduced pressure to provide as the residue a foamed product of 2β-(4-carboxy-1,2,3-triazole-1-yl)methyl-2α-methylpenam-3α-carboxylic acid.

A 2 g quantity of the 2β-(4-carboxy-1,2,3-triazole-1-yl)methyl-2α-methylpenam-3α-carboxylic acid was dissolved in 20 ml of butanol. To the solution was added a solution of potassium 2-ethyl hexanoate in butanol, and the mixture was stirred awhile at room temperature. The precipitate was filtered to give 2.0 g of white solids having a melting point of over 178°C (decomposition).

Infrared absorption spectrum ($D_2O$) ν max ($cm^{-1}$):
1780, 1610

Nuclear magnetic resonance spectrum ($D_2O$) δ (ppm):
1.47 (3Hs, s), 3.49 (1H, dd), 3.77 (1H, dd), 4.53 (1H, s), 5.0—5.1 (1H, m), 5.16 (1H, d), 5.41 (1H, d), 8.47 (1H, s).

Example 13

Preparation of dipotassium 2β-(5-carboxy-1,2,3-triazole-1-yl)methyl-2α-methylpenam-3α-carboxylate-1,1-dioxide (Compound 17)

White solid of the contemplated compound with a melting point of over 175°C (decomposition) was prepared in the same manner as in Example 12 by using p-nitrobenzyl 2α-methyl-2β-[5-(p-nitrobenzyloxycarbonyl-1,2,3-triazole-1-yl]methylpenam-3α-carboxylate-1,1-dioxide.

Infrared absorption spectrum (KBr) ν max ($cm^{-1}$):

1780, 1610

Nuclear magnetic resonance spectrum ($D_2O$) δ (ppm):

1.40 (3H, s), 3.43 (1H, dd), 3.71 (1H, dd), 4.58 (1H, s), 4.9—5.1 (1H, m), 5.36 (1H, d), 5.93 (1H, d), 8.04 (1H, s).

Example 14

Preparation of benzhydryl 2β-(4-carboxy-1,2,3-triazole-1-yl)methyl-2α-methylpenam-3α-carboxylate-1,1-dioxide (Compound 18)

A 0.5 g quantity of benzhydryl 2β-azidomethyl-2α-methylpenam-3α-carboxylate-1,1-dioxide and 0.083 g of acetylenecarboxylic acid were stirred in 2 ml of dichloromethane at room temperature under nitrogen atmosphere for 24 hours. The solvent was removed by distillation at reduced pressure and to the residual oil was added benzene. The insolubles were filtered off and to the residue was added hexane to deposit crystals which were collected by filtration. Thus there was produced 0.23 g of white crystals which melt at 120 to 121°C.

Infrared absorption spectrum (KBr) ν max ($cm^{-1}$):

1805, 1745

Nuclear magnetic resonance spectrum δ (ppm):

1.07 (3H, s), 3.2—3.8 (2H, m), 4.5—4.7 (1H, m), 4.69 (1H, s), 5.12 (2H, bs), 7.02 (1H, s), 7.1—7.6 (10H, m), 8.33 (1H, s).

Example 15

Preparation of disodium 2β-(4-carboxy-1,2,3-triazole-1-yl)methyl-2α-methylpenam-3α-carboxylate-1,1-dioxide (Compound 19)

Hydrogenation was conducted in 10 ml of ethyl acetate and 10 ml of water at room temperature for 30 minutes by using 49 mg of benzhydryl 2β-(4-carboxy-1,2,3-triazole-1-yl)methyl-2α-methylpenam-3α-carboxylate-1,1-dioxide. 15 ml of 10% palladium charcoal and 24 mg of sodium hydrogencarbonate. The aqueous layer was separated from the reaction mixture and washed with ethyl acetate, and was purified with an MCI gel, CHP—20P (product of Mitsubishi Kasei Co., Ltd., Japan). After freeze-drying, there was obtained a white amorphous product having a melting point of 220 to 250°C (decomposition).

The values of the infrared absorption spectrum and nuclear magnetic resonance spectrum of the compound thus obtained were similar to those of Compound 16 prepared in Example 12.

Example 16

Preparation of benzhydryl 2α-methyl-2β-(4-trimethylsilyl-1,2,3-triazole-1-yl)methylpenam-3α-carboxylate-1,1-dioxide (Compound 20)

A 150 mg quantity of benzhydryl 2β-azodimethyl-2α-methylpenam-3α-carboxylate-1,1-dioxide was reacted in a sealed reactor with 300 mg of trimethylsilylacetylene at 90 to 95°C for 20 hours. The reaction mixture was concentrated at reduced pressure, giving 170 mg of white crystals which melt at 172 to 175°C.

Infrared absorption spectrum (KBr) ν max ($cm^{-1}$):

1805, 1755

Nuclear magnetic resonance spectrum ($CDCl_3$) δ (ppm):

0.32 (9H, s), 1.05 (3H, s), 3.3—3.7 (2H, m), 4.5—4.7 (1H, m), 4.65 (1H, s), 5.08 (2H, AB-q), 7.00 (1H, s), 7.3—7.5 (10H, m), 7.67 (1H, s).

Example 17

Preparation of benzhydryl 2α-methyl-2β-(1,2,3-triazole-1-yl)methylpenam-3α-carboxylate-1,1-dioxide (Compound 21)

A 133 mg quantity of benzhydryl 2α-methyl-2β-(4-trimethylsilyl-1,2,3-triazole-1-yl)methylpenam-3α-carboxylate-1,1-dioxide, 3.26 g of 18-crown-6(1,4,7,10,13,16-hexaoxacyclooctadecane) and 15.8 mg of potassium fluoride were stirred in 0.7 ml of N,N-dimethylformamide at 50 to 60°C for 5.5 hours. The reaction mixture was poured into excess iced water and the mixture was extracted a few times with ethyl acetate. The ethyl acetate extracts were collected and dried over magnesium sulfate. The solvent was distilled off at reduced pressure and the residue was purified by column chromatography on silica gel, whereby a white product was given which has a melting point of 206 to 208°C (decomposition).

Infrared absorption spectrum (KBr) ν max ($cm^{-1}$):

1800, 1760

Nuclear magnetic resonance spectrum ($CDCl_3$) δ (ppm):

1.05 (3H, s), 3.3—3.7 (2H, m), 4.5—4.7 (1H, m), 4.65 (1H, s), 5.10 (2H, AB-q), 7.00 (1H, s), 7.3—7.5 (10H, m), 7.73 (1H, s).

Example 18

Preparation of benzhydryl 2α-methyl-2β-(1,2,3-triazole-1-yl)methylpenam-3α-carboxylate-1,1-dioxide (Compound 21)

A 500 mg quantity of benzhydryl 2β-azidomethyl-2α-methylpenam-3α-carboxylate-1,1-dioxide, 335 mg of trimethylsilylacetylene and 2 ml of methylene chloride were reacted in a sealed reactor at 95°C for 20 hours. The reaction mixture was concentrated at reduced pressure and the residue was purified by column chromatography on silica gel to provide white solids having a melting point of 203 to 204°C (decomposition). Fast atomic bombardment mass spectrum method.

$$m/e = 467 (M^+)$$

The values of the infrared absorption spectrum and nuclear magnetic resonance spectrum on the compound thus obtained were identical with those of Compound 21 obtained in Example 17.

Example 19

Preparation of benzhydryl 2α-methyl-2β-(1,2,3-triazole-1-yl)methylpenam-3α-carboxylate-1,1-dioxide (Compound 21)

A 200 mg quantity of benzhydryl 2β-azidomethyl-2α-methylpenam-3α-carboxylate-1,1-dioxide was reacted with with 10 ml of vinyl acetate in a sealed reactor at 100 to 110°C for 30 hours. The reaction mixture was concentrated at reduced pressure. The residue was crystallized with cooled chloroform.

The white crystals thus obtained were found to have a melting point (decomposition) and the values of the nuclear magnetic resonance spectrum which were all identical with the values of Compound 21 obtained in Example 17.

Example 20

Preparation of sodium 2α-methyl-2β-(1,2,3-triazole-1-yl)-methylpenam-3α-carboxylate-1,1-dioxide (Compound 22)

Hydrogenation was conducted in 10 ml of ethyl acetate and 10 ml of water at room temperature for 30 minutes by using 45 mg of benzhydryl 2α-methyl-2β-(1,2,3-triazole-1-yl)-methylpenam-3α-carboxylate-1,1-dioxide, 15 mg of 10% palladium charcoal and 16 mg of sodium hydrogencarbonate. The aqueous layer was separated from the reaction mixture and washed once with ethyl acetate. The aqueous solution was then purified with an MCl gel, CHP—20P (product of Mitsubishi Kasei Co., Ltd., Japan). After freeze-drying, there was obtained an amorphous product with a melting point of over 170°C (decomposition).

Infrared absorption spectrum (KBr) ν max ($cm^{-1}$):

3430, 1780, 1630

Nuclear magnetic resonance spectrum ($D_2O$) δ (ppm):

1.41 (3H, s), 3.45 (1H, dd), 3.72 (1H, dd), 4.48 (1H, s), 4.96—5.10 (1H, m), 5.25 (2H, AB-q), 7.85 (1H, d), 8.13 (1H, d)

Example 21

Preparation of p-nitrobenzyl 2α-methyl-2β-(1,2,3-triazole-1-yl)-methylpenam-3α-carboxylate-1,1-dioxide (Compound 23)

A 1.02 g quantity of p-nitrobenzyl 2β-azido-methyl-2α-methylpenam-3α-carboxylate-1,1-dioxide was reacted with 50 ml of vinyl acetate in a sealed reactor at 100 to 110°C for 3 hours. The reaction mixture was concentrated at reduced pressure and the residue was purified by column chromatography on silica gel giving 0.73 g of the contemplated compound in amorphous form in 67% yield which melts at 182 to 184°C

Infrared absorption spectrum (KBr) ν max ($cm^{-1}$):

1800, 1760

Nuclear magnetic resonance spectrum ($CDCl_3$) δ (ppm):

1.26 (3H, s), 3.5—3.6 (2H, m), 4.66 (1H, s), 4.6—4.7 (1H, m), 5.07 (2H, s), 5.36 (2H, s), 7.61 (2H, d), 7.74 (1H, d), 7.80 (1H, d), 8.28 (2H, d).

Example 22

Preparation of sodium 2α-methyl-2β-(4-trimethylsilyl-1,2,3-triazole-1-yl)-methylpenam-3α-carboxylate-1,1-dioxide (Compound 24)

Hydrogenation was performed in 15 ml of ethyl acetate and 15 ml of water at room temperature for 30 minutes by using 200 mg of benzhydryl 2α-methyl-2β-(4-trimethylsilyl-1,2,3-triazole-1-yl)-methylpenam-3α-carboxylate-1,1-dioxide, 50 mg of 10% palladium charcoal and 98 mg of sodium hydrogencarbonate. The aqueous layer was removed from the reaction mixture and washed once with ethyl acetate. The aqueous solution was purified with an MCl gel, CHP—20P (product of Mitsubishi Kasei Co., Ltd., Japan). After freeze-drying, there was obtained an amorphous product having a melting point of over 170°C (decomposition).

Infrared absorption spectrum (KBr) ν max ($cm^{-1}$):
3440, 1780, 1630
Nuclear magnetic resonance spectrum ($D_2O$) δ (ppm):
0.32 (9H, s), 1.38 (3H, s), 3.1—3.7 (2H, m), 4.46 (1H, s), 4.9—5.0 (1H, m), 5.23 (2H, AB-q), 8.16 (1H, s)

Example 23

Preparation of benzhydryl 2β-(4,5-dimethoxycarbonyl-1,2,3-triazole-1-yl)-methyl-2α-methylpenam-3α-carboxylate-1,1-dioxide (Compound 25)

A 0.870 g quantity of benzhydryl 2β-azidomethyl-2α-methylpenam-3α-carboxylate-1,1-dioxide and 0.618 g of dimethylacetylenedicarboxylate in 15 ml of benzene were refluxed with stirring under nigrogen atmosphere for 18 hours. The solvent was distilled off and the residue was subjected to column chromatography on silica gel using a mixture of ethyl acetate and chloroform in the ratio of 1:3. There was obtained 0.495 g of the contemplated compound as pale yellow crystals with a melting point of 75 to 77°C in 44% yield.

Infrared absorption spectrum (KBr) ν max ($cm^{-1}$):
1800, 1735
Nuclear magnetic resonance spectrum ($CDCl_3$) δ (ppm):
1.20 (3H, s), 3.48 (2H, t), 3.97 (3H, s), 3.98 (3H, s), 4.59 (1H, m), 4.95 (1H, s), 5.26 (2H, s), 6.97 (1H, s), 7.36 (10H, s)

Example 24

Preparation of sodium 2β-(4,5-dimethoxycarbonyl-1,2,3-triazole-1-yl)-methyl-2α-methylpenam-3α-carboxylate-1,1-dioxide (Compound 26)

Hydrogenation was conducted at room temperature by using 100 ml of tetrahydrofuran, 100 ml of water, 116 mg of benzhydryl 2β-(4,5-dimethoxycarbonyl-1,2,3-triazole-1-yl)-methyl-2α-methylpenam-3α-carboxylate-1,1-dioxide, 58 mg of 10% palladium charcoal and 17 mg of sodium hydrogencarbonate. After completion of absorption of hydrogen, the reaction liquid was filtered and the tetrahydrofuran was removed from the filtrate by distillation at reduced pressure. The residue was washed with chloroform and the aqueous solution was concentrated at reduced pressure. The concentrate was subjected to column chromatography using an MCI gel, CHP—20P (product of Mitsubishi Kasei Co., Ltd., Japan) and gradiently eluted with a water—10% acetone water mixture. The eluate thus obtained was freeze-dried to provide 53 mg of the contemplated product as white powder in 60% yield. The white powder decomposed at a temperature of over 165°C.

Infrared absorption spectrum (KBr) ν max ($cm^{-1}$):
1785, 1735, 1630
Nuclear magnetic resonance spectrum ($D_2O$) δ (ppm):
1.41 (3H, s), 3.40 (1H, dd), 3.80 (1H, dd), 3.98 (3H, s), 4.05 (3H, s), 4.51 (1H, s), 5.03 (1H, dd), 5.48 (2H, d)

Example 25

Preparation of 2β-(4,5-dimethoxycarbonyl-1,2,3-triazole-1-yl)methyl-2α-methylpenam-3α-carboxylic acid-1,1-dioxide (Compound 27)

Hydrogenation was carried out in 100 ml of tetrahydrofuran and 100 ml of water at room temperature by using 116 mg of benzhydryl 2β-(4,5-dimethoxycarbonyl-1,2,3-triazole-1-yl)methyl-2α-methylpenam-3α-carboxylate-1,1-dioxide, 58 mg of 10% palladium charcoal and 17 mg of sodium hydrogencarbonate. After completion of absorption of hydrogen, the reaction liquid was filtered and the tetrahydrofuran was distilled off from the filtrate at reduced pressure. The residue was washed with chloroform. After adjusting its pH to 1.5 to 1.7 with diluted hydrochloric acid, the aqueous solution was washed with chloroform, extracted with ethyl acetate and dried over magnesium sulfate. The ethyl acetate was distilled off at reduced pressure, and the residue was dissolved in water. The solution was freeze-dried to provide 83 mg of amorphous product in 76% yield which has a melting point of 135 to 145°C (decomposition).

Infrared absorption spectrum (KBr) ν max ($cm^{-1}$):
1805, 1735
Nuclear magnetic resonance spectrum ($CDCl_3$ + DMSO-$d_6$) δ (ppm):
1.55 (3H, s), 3.2—3.6 (2H, m), 3.96 (3H, s), 4.02 (3H, s), 4.68 (1H, s), 4.6—4.8 (1H, m), 5.35 (2H, s)

Example 26

Preparation of chloromethyl 2β-(4,5-dimethoxycarbonyl-1,2,3-triazole-1-yl)-methyl-2α-methylpenam-3α-carboxylate-1,1-dioxide (Compound 28)

A 1.25 g quantity of sodium hydrogencarbonate and 0.133 g of tetrabutylammonium hydrogensulfate were added with stirring at less than 10°C to 1.61 g of 2β-(4,5-dimethoxycarbonyl-1,2,3-triazole-1-yl)-methyl-2α-methylpenam-3α-carboxylic-1,1-dioxide, 8 ml of dichloromethane and 8 ml of water. To the mixture was dropwise added at the same temperature 0.74 g of chloromethyl chlorosulfonate. The mixture was stirred at room temeperature for 30 minutes. The organic layer was separated, washed once with

water and dried over magnesium sulfate. The solvent was removed by distillation at reduced pressure to obtain as the residue 1.5 g of amorphous product in 83% yield.

Infrared absorption spectrum (KBr) ν max ($cm^{-1}$):

1805, 1735

Nuclear magnetic resonance spectrum ($CDCl_3$) δ (ppm):

1.55 (3H, s), 3.2—3.8 (2H, m), 3.99 (3H, s), 4.03 (3H, s), 4.6—4.8 (1H, m), 5.01 (1H, s), 5.32 (2H, d), 5.62 (1H, d), 5.81 (1H, d)

Example 27

Preparation of iodomethyl 2β-(4,5-dimethoxycarbonyl-1,2,3-triazole-1-yl)methyl-2α-methylpenam-3α-carboxylate-1,1-dioxide (Compound 29)

A 1.05 g quantity of chloromethyl 2β-(4,5-dimethoxycarbonyl-1,2,3-triazole-1-yl)methyl-2α-methylpenam-3α-carboxylate-1,1-dioxide, 0.52 g of sodium iodide and 1.8 ml of acetone were stirred at room temperature for 18 hours. To the reaction mixture was added 1.5 ml of water and the resulting mixture was adjusted to a pH of 7 to 8 with an aqueous solution of sodium hydrogencarbonate. Thereto was added 1.5 ml of water and the mixture was decolorized with an aqueous solution of 0.5 M sodium thiosulfate. The mixture was extracted with dichloromethane, washed with water and dried over magnesium sulfate. The solvent was distilled off at reduced pressure to produce 1.1 g of amorphous product in 78% yield.

Infrared absorption spectrum (KBr) ν max ($cm^{-1}$):

1800, 1730

Nuclear magnetic resonance spectrum ($CDCl_3$) δ (ppm):

1.56 (3H, s), 3.2—3.8 (2H, m), 3.99 (3H, s), 4.03 (3H, s), 4.6—4.8 (1H, m), 4.97 (1H, s), 5.30 (2H, d), 5.85 (1H, d), 5.96 (1H, d)

Example 28

Preparation of p-nitrobenzyl 2β-(4,5-dimethoxycarbonyl-1,2,3-triazole-1-yl)-methyl-2α-methylpenam-3α-carboxylate-1,1-dioxide (Compound 30)

A 3.5 g quantity of p-nitrobenzyl 2β-azidomethyl-2α-methylpenam-3α-carboxylate-1,1-dioxide and 4.8 g of dimethylacetylenedicarboxylate in 80 ml of absolute benzene were refluxed under nitrogen atmosphere for 18 hours. The solvent was distilled off at reduced pressure to provide 4.7 g of the contemplated compound.

Infrared absorption spectrum (KBr) ν max ($cm^{-1}$):

1805, 1735

Nuclear magnetic resonance spectrum ($CDCl_3$) δ (ppm):

1.43 (3H, s), 3.2—3.4 (2H, m), 3.96 (3H, s), 3.99 (3H, s), 4.64—4.76 (1H, m), 4.98 (1H, s), 5.04—5.44 (4H, m), 7.56 (2H, d), 8.23 (2H, d)

Example 29

Preparation of p-nitrobenzyl 2β-(4,5-diethoxycarbonyl-1,2,3-triazole-1-yl)methyl-2α-methylpenam-3α-carboxylate-1,1-dioxide (Compound 31)

The foregoing compound was prepared in the same manner as in Example 23 with the exception of using as the starting material p-nitrobenzyl 2β-azidomethyl-2α-methylpenam-3α-carboxylate-1,1-dioxide.

Infrared absorption spectrum (KBr) ν max ($cm^{-1}$):

1805, 1735

Nuclear magnetic resonance spectrum ($CDCl_3$) δ (ppm):

1.33—1.48 (9H, m), 3.30—3.72 (2H, m), 4.42 (2H, q), 4.45 (2H, q), 4.64—4.71 (1H, m), 5.02 (1H, s), 5.22—5.40 (4H, m), 7.55 (2H, d), 8.24 (2H, d)

Example 30

Preparation of 2β-(4,5-diethoxycarbonyl-1,2,3-triazole-1-yl)methyl-2α-methylpenam-3α-carboxylic acid-1,1-dioxide (Compound 32) and sodium salt thereof (Compound 33)

Hydrogenation was conducted at an initial pressure of 3 atm. by using 3.5 g of p-nitrobenzyl 2β-(4,5-diethoxycarbonyl-1,2,3-triazole-1-yl)methyl-2α-methylpenam-3α-carboxylate-1,1-dioxide, 1.1g of sodium hydrogencarbonate, 0.6 g of 10% palladium charcoal, 90 ml of ethyl acetate and 90 ml of water. In 1 hour, the reaction mixture was sedimented and the aqueous layer was separated. The aqueous solution was washed twice with ether and its pH was adjusted to 1.7 with diluted hydrochloric acid. The aqueous solution was extracted with ethyl aetate and dried over magnesium sulfate. The solvent was removed by distillation at reduced pressure. Thus there was obtained 2.2 g of 2β-(4,5-diethoxycarbonyl-1,2,3-triazole-1-yl)methyl-2α-methylpenam-3α-carboxylic acid-1,1-dioxide in 83% yield in amorphous form.

A 170 mg quantity of 2β-(4,5-diethoxycarbonyl-1,2,3-triazole-1-yl)methyl-2α-methylpenam-3α-carboxylic acid-1,1-dioxide was dissolved in a solution of 84 mg of sodium hydrogencarbonate in 10 ml of water. The solution was purified with an MCI gel, CHP—20P (product of Mitsubishi Kasei Co, Ltd., Japan). The liquid thus obtained was freeze-dried and there was prepared 130 mg of sodium 2β-(4,5-diethoxycarbonyl-1,2,3-triazole-1-yl)methyl-2α-methylpenam-3α-carboxylate-1,1-dioxide in amorphous

form having a melting point of 155 to 160°C (decomposition) in 73% yield.

Infrared absorption spectrum (KBr) ν max ($cm^{-1}$):

1785, 1735, 1630

Nuclear magnetic resonance spectrum ($D_2O$) δ (ppm):

1.32—1.49 (9H, m), 3.34—3.82 (2H, m), 4.35—4.65 (5H, m), 5.01—5.07 (1H, m), 5.47 (2H, s)

Example 31

Preparation of: p-nitrobenzyl 2β-(4,5-di-n-butoxycarbonyl-1,2,3-triazole-1-yl)methyl-2α-methylpenam-3α-carboxylate-1,1-dioxide (Compound 34); p-nitrobenzyl 2β-(4,5-di-isobutoxycarbonyl-1,2,3-triazole-1-yl)methyl-2α-methylpenam-3α-carboxylate-1,1-dioxide (Compound 35); p-nitrobenzyl 2β-(4,5-di-n-tetradecyloxycarbonyl-1,2,3-triazole-1-yl)methyl-2α-methylpenam-3α-carboxylate-1,1-dioxide (Compound 36); p-nitrobenzyl 2β-(4,5-di-n-octadecyloxycarbonyl-1,2,3-triazole-1-yl)methyl-2α-methylpenam-3α-carboxylate-1,1-dioxide (Compound 37); p-nitrobenzyl 2β-[4,5-di-n-(p-nitrobenzyloxy)carbonyl-1,2,3-triazole-1-yl]methyl-2α-methylpenam-3α-carboxylate-1,1-dioxide (Compound 38).

The foregoing compounds were prepared in the same manner as in Example 29.

Compound 34

Infrared absorption spectrum (NaCl) ν max ($cm^{-1}$)

1805, 1730

Nuclear magnetic resonance spectrum ($CDCl_3$) δ (ppm):

0.96 (6H, t), 1.43 (3H, s), 1.2—2.0 (8H, m), 3.3—3.7 (2H, m), 4.2—4.5 (4H, m), 4.6—4.7 (1H, m), 5.02 (1H, s), 5.0—5.4 (4H, m), 7.54 (2H, d), 8.24 (2H, d)

Compound 35

Infrared absorption spectrum (NaCl) ν max ($cm^{-1}$):

1805, 1735

Nuclear magnetic resonance spectrum ($CDCl_3$) δ (ppm):

0.98 (6H, d), 1.00 (6H, d), 1.43 (3H, s), 1.8—2.3 (2H, m), 3.3—3.7 (2H, m), 4.15 (4H, d), 4.5—4.7 (1H, m), 5.02 (1H, s), 5.0—5.4 (4H, m), 7.55 (2H, d), 8.24 (2H, d)

Compound 36

Infrared absorption spectrum (KBr) ν max ($cm^{-1}$):

1800, 1730

Nuclear magnetic resonance spectrum ($CDCl_3$) δ (ppm):

0.70—1.90 (57H m), 3.44—3.60 (2H, m), 4.22—4.45 (4H, m), 4.58—4.70 (1H, m), 5.03 (1H, s), 4.95—5.40 (4H, m), 7.54 (2H, d), 8.24 (2H, d)

Compound 37

Infrared absorption spectrum (KBr) ν max ($cm^{-1}$):

1800, 1730

Nuclear magnetic resonance spectrum ($CDCl_3$) δ (ppm):

0.70—1.90 (73H, m), 3.44—3.60 (2H, m), 4.22—4.45 (4H, m), 4.54—4.68 (1H, m), 5.03 (1H, s), 4.80—5.40 (4H, m), 7.53 (2H, d), 8.24 (2H, d)

Compound 38

Infrared absorption spectrum (KBr) ν max ($cm^{-1}$):

1800, 1735

Nuclear magnetic resonance spectrum ($CDCl_3$) δ (ppm):

1.46 (3H, s), 3.42—3.56 (2H, m), 4.58—4.70 (1H, m), 4.88 (1H, s), 5.31 (4H, s), 5.46 (4H, s), 7.42—7.66 (6H, m), 8.05—8.30 (6H, m)

Example 32

Preparation of tri-sodium 2β-(4,5-dicarboxy-1,2,3-triazole-1-yl)methyl-2α-methylpenam-3α-carboxylate-1,1-dioxide (Compound 39)

The above compound with a melting point of over 203°C (decomposition) was produced in the same manner as in Example 4 from Compound 38.

Infrared absorption spectrum (KBr) ν max ($cm^{-1}$):

1780, 1720

Nuclear magnetic resonance spectrum ($D_2O$) δ (ppm):

1.42 (3H, s), 3.25—3.82 (2H, m), 4.50 (1H, s), 4.95—5.04 (1H, m), 5.52 (2H, dd)

The compounds obtained in some of the examples were checked for β-lactamase inhibitory activity and antibacterial activity.

(1) Test for β-lactamase inhibitory activity

The inhibitory activity against penicillinase (β-lactamase) from Bacillus SP was measured by

microiodometry [Tanpakushitsu Kakusan Koso (Protein Nucleic Acid Enzyme), vol. 23, No. 5, pp 391—400 (1978)] using a penicillin G as a substrate. Table 1 given below shows the results.

TABLE 1

| Compound | 50% Inhibitory Concentration |
|---|---|
| Compound 7 | $5.4 \times 10^{-8}M$ |
| „ 11 | $3.4 \times 10^{-7}M$ |
| „ 12 | $4.9 \times 10^{-8}M$ |
| „ 13 | $3.0 \times 10^{-7}M$ |
| „ 16 | $6.0 \times 10^{-7}M$ |
| „ 17 | $1.7 \times 10^{-6}M$ |
| „ 22 | $6.9 \times 10^{-7}M$ |
| „ 24 | $5.1 \times 10^{-7}M$ |
| „ 39 | $2.3 \times 10^{-6}M$ |

Further, the sodium 2β-(4,5-dimethoxycarbonyl-1,2,3-triazole-1-yl)methyl-2α-methylpenam-3α-carboxylate-1,1-dioxide (Compound 26) obtained in Example 24 was tested for β-lactamase inhibitory activity against penicillinase (β-lactamase) from Bacillus SP by pH State method ("Journal Pharmaceutical Science" published in 1972, vol. 61, No. 10, pp 1954 to 1958) using as a susbtrate a penicillin G, and was found to have 50% β-lactamase inhibitory concentration of $3 \times 10^{-7}M$.

(2) Test for antibacterial activity

*1) Effects by ampicillin as combined with the present compound*

The compounds of the present invention and ampicillin, each singly used, were checked for minimal inhibitory concentration (MIC) against the bacteria listed in Table 2 given below by micro-broth dilution method ("American Journal Clinical Pathology" published in 1980, vol. 73, No. 3, pp 374 to 379). The MIC of ampicillin as combined with the present compound (10 μg/ml) was measured against the same bacteria. In the method, the bacteria cultivated in Mueller Hinton Broth (product of DIFCO) and diluted to $10^7$ CFU/ml were inoculated into the same medium containing ampicillin and the present compound in a specific concentration, and incubated at 37°C for 20 hours. Thereafter the growth of the microorganisms was observed to determine the minimal inhibitory concentration (MIC) for rendering the inoculated medium free from turbidity. The present compounds, singly used, turned out to be all more than 25 μg/ml in MIC. The bacteria as used in the test were those capable of producing β-lactamase, among which the bacteria marked * in the table are those collected from the living body of human hosts and the others are a stock culture.

In Table 2, the present compounds are shown by the compound number.

TABLE 2

| Test Bacteria | MIC (µg/ml) | | | | | | | | |
|---|---|---|---|---|---|---|---|---|---|
| | | Present Compound (combined with ampicillin) | | | | | | | |
| | Ampicillin (singly used) | 7 | 11 | 12 | 13 | 16 | 17 | 22 | 24 |
| S.aureus S—54 | 25 | 0.1 | 0.2 | 0.2 | 0.2 | 0.2 | 0.78 | 0.2 | 0.78 |
| S.aureus ATCC 90124 | 25 | 0.1 | 0.2 | 0.2 | 0.2 | 0.2 | 0.78 | 0.1 | 0.39 |
| E.coli TH—13* | 400 | 6.25 | 25 | 3.13 | 6.25 | 6.25 | 0.05 | 3.13 | 100 |
| E.coli TH—397* | 400 | 6.25 | 12.5 | 3.13 | 6.25 | 3.13 | 6.25 | 6.25 | 50 |
| P.Mirabilis 121 | >400 | 1.56 | 0.78 | 0.78 | 0.78 | 0.78 | 0.39 | 0.78 | 25 |
| P.vulgaris IID OX—19 | 100 | 0.78 | 0.78 | 0.39 | 0.39 | 1.56 | 1.56 | 0.78 | 1.56 |
| S.marcescens TH—05* | >400 | 12.5 | 25 | 12.5 | 25 | 6.25 | 1.56 | 3.13 | 100 |

The same test as above was carried out to determine the antibacterial activity of the sodium 2β-(4,5-dimethoxycarbonyl-1,2,3-triazole-1-yl)methyl-2α-methylpenam-3α-carboxylate-1,1-dioxide (Compound 26) obtained in Example 24. Table 3 given below shows the results.

TABLE 3

| Test bacteria | Amount of inoculate (CFU/ml) | MIC (µg/ml) | | |
|---|---|---|---|---|
| | | Ampicillin | Comp. 26 | Ampicillin as combined with Comp. 26 |
| S.aureus S—54 | $10^7$ | 100 | >50 | 0.39 |
| S.aureus ATCC 90124 | $10^7$ | 50 | >50 | ≦0.2 |
| E.coli TH—13* | $10^7$ | 400 | >50 | 25 |
| E.coli TH—34* | $10^7$ | 800 | >50 | 50 |
| P.mirabilis 121—1 | $10^7$ | 800 | >50 | 1.56 |
| P.vulgaris IID OX—19 | $10^7$ | 25 | 25 | ≦0.2 |
| S.marcescens TH—05* | $10^7$ | 800 | >50 | 50 |

*2) Effects by antibiotics as combined with the present compound*

The compounds of the present invention, ampicillin, mecillinam, piperacillin and cephalexin, each singly used, were also tested for minimal inhibitory concentration against 30 strains of coliform bacilli collected from the living body of humans. The MIC of each antibiotic as combined with the present compound (10 μg/ml) was likewise measured. Table 4 to 7 indicate the results in which $MIC_{50}$ and $MIC_{70}$ indicate the minimal inhibitory concentration for inhibiting the growth of 50% and 70% respectively of the strains. The MICs of the present compounds singly used were all more than 25 μg/ml.

TABLE 4

| 30 Strains of coriform bacilli | Ampicillin singly used | Present compound as combined with ampicillin | | | | |
|---|---|---|---|---|---|---|
| | | Comp. 7 | Comp. 11 | Comp. 16 | Comp. 17 | Comp. 22 |
| $MIC_{50}$ (μg/ml) | 400 | 6.25 | 50 | 6.25 | 25 | 3.13 |
| $MIC_{70}$ (μg/ml) | >400 | 50 | 100 | 6.25 | 100 | 6.25 |

TABLE 5

| 30 Strains of coriform bacilli | Mecillinam singly used | Present compound as combined with mecillinam | | | | |
|---|---|---|---|---|---|---|
| | | Comp. 7 | Comp. 11 | Comp. 16 | Comp. 17 | Comp. 22 |
| $MIC_{50}$ (μg/ml) | 3.13 | 0.2 | 0.2 | 0.1 | 0.05 | 0.1 |
| $MIC_{70}$ (μg/ml) | 12.5 | 0.39 | 0.39 | 0.1 | 0.39 | 0.2 |

TABLE 6

| 30 Strains of coriform bacilli | Piperacillin singly used | Present compound as combined with piperacillin | | | | |
|---|---|---|---|---|---|---|
| | | Comp. 7 | Comp. 11 | Comp. 16 | Comp. 17 | Comp. 22 |
| $MIC_{50}$ (μg/ml) | 50 | 1.56 | 6.25 | 1.56 | 6.25 | 1.56 |
| $MIC_{70}$ (μg/ml) | 200 | 6.25 | 25 | 3.13 | 50 | 1.56 |

TABLE 7

| 30 Strains of coriform bacilli | Cephalexin singly used | Present compound as combined with cephalexin | | | | |
|---|---|---|---|---|---|---|
| | | Comp. 7 | Comp. 11 | Comp. 16 | Comp. 17 | Comp. 22 |
| $MIC_{50}$ (μg/ml) | 25 | 12.5 | 12.5 | 6.25 | 3.13 | 12.5 |
| $MIC_{70}$ (μg/ml) | 100 | 100 | 100 | 25 | 12.5 | 50 |

Given below are examples of preparation of the present antibacterial compositions.

Preparation Example 1

| | |
|---|---|
| Ampicillin | 200 mg |
| Compound 22 | 200 mg |
| Lactose | 100 mg |
| Crystalline cellulose | 57 mg |
| Magnesium stearate | 3 mg |
| Total | 560 mg |
| | (amount per capsule) |

The above ingredients are formulated in the proportions listed above into a capsule.

Preparation Example 2

| | |
|---|---|
| Amoxycillin | 100 mg |
| Compound 16 | 70 mg |
| Lactose | 330 mg |
| Corn starch | 490 mg |
| Hydroxypropyl methyl cellulose | 10 mg |
| Total | 1000 mg |
| | (amount per dose) |

The above ingredients are formulated in the proportions listed above into granules.

Preparation Example 3

| | |
|---|---|
| Pivmecillinam | 70 mg |
| Compound 17 | 70 mg |
| Lactose | 33 mg |
| Crystalline cellulose | 15 mg |
| Magnesium stearate | 3 mg |
| Talc | 4 mg |
| Corn starch | 15 mg |
| Hydroxypropyl methyl cellulose | 10 mg |
| Total | 220 mg |
| | (amount per tablet) |

The above ingredients are formulated in the proportions listed above into a tablet.

Preparation Example 4

| | |
|---|---|
| Compound 22 | 120 mg |
| Hydroxypropyl cellulose | 3 mg |
| Corn starch | 25 mg |
| Magnesium stearate | 2 mg |
| Total | 150 mg |
| | (amount per tablet) |

The above ingredients are formulated in the proportions listed above into a tablet.

**Claims**

1. A penicillin derivative represented by the following formula, and a pharmaceutically acceptable salt thereof and an ester thereof:

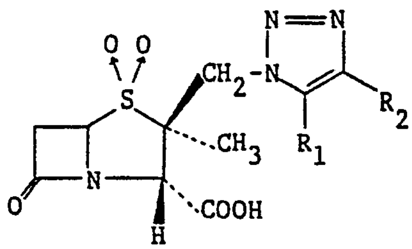

(I)

wherein $R_1$ and $R_2$, which may be the same or different, represent hydrogen atoms, tri $C_{1-6}$ alkylsilyl groups, carboxyl groups, or carboxyl groups forming a pharmaceutically acceptable salt, or esterified carboxyl groups.

2. A derivative according to claim 1 in which one of $R_1$ and $R_2$ is hydrogen and the other is an esterified carboxyl group.

3. A derivative according to claim 1 in which $R_1$ and $R_2$ are both esterified carboxyl groups.

4. A derivative according to claim 1 in which one of $R_1$ and $R_2$ is hydrogen and the other is hydrogen carboxyl or a carboxyl group forming a pharmaceutically acceptable salt.

5. A derivative according to claim 1 in which any one of $R_1$ and $R_2$ is tri $C_{1-6}$ alkylsilyl.

6. A derivative according to any one of claims 1, 2 and 3 in which the esterified carboxyl group is alkoxycarbonyl having 1—18 carbon atoms in the alkyl group.

7. A pharmaceutically acceptable salt of the derivative according to claim 1 which salt is an alkali metal salt, alkaline earth metal salt, organic amine salt, basic amino acid salt or ammonium salt.

8. A pharmaceutically acceptable ester of the derivative according to claim 1 which ester is readily hydrolyzed *in vivo* to produce a free acid.

9. A process for preparing a penicillin derivative represented by the following formula and a pharmaceutically acceptable salt thereof and an ester thereof:

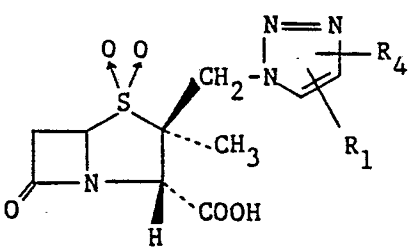

wherein $R_1$ represents hydrogen, tri $C_{1-6}$ alkylsilyl, carboxyl, carboxyl forming a pharmaceutically acceptable salt or esterified carboxyl, and $R_4$ represents a tri $C_{1-6}$ alkylsilyl group, a carboxyl group, a carboxyl group forming a pharmaceutically acceptable salt, or an esterified carboxyl group, the process comprising reacting a compound of the formula

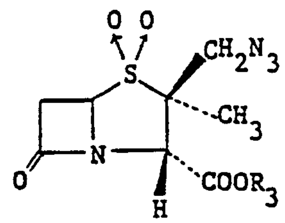

wherein $R_3$ represents a penicillin carboxyl-protecting group with an acetylene derivative of the formula

$$R_1C{\equiv}CR_4$$

wherein $R_1$ and $R_4$ are as defined above, and, when required, carrying out any of de-esterification, esterification subsequent to de-esterification, ester interchange reaction and salt-forming reaction.

10. A process for preparing a penicillin derivative represented by the following formula, and a pharmaceutically acceptable salt thereof and an ester thereof:

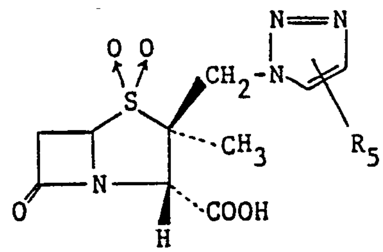

wherein $R_5$ represents hydrogen, carboxyl, carboxyl forming a pharmaceutically acceptable salt or esterified carboxyl, the process comprising reacting a compound of the formula

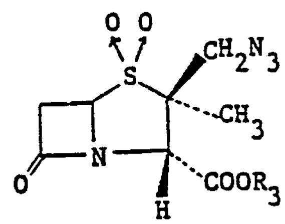

wherein $R_3$ represents a penicillin carboxyl-protecting group with an acetylene derivative of the formula

$$R_1'C{\equiv}CR_4'$$

wherein $R_1'$ and $R_4'$ each represents hydrogen, tri $C_{1-6}$ alkylsilyl, carboxyl, a carboxyl group forming a pharmaceutically acceptable salt or an esterified carboxyl group, provided that at least one of $R_1'$ and $R_4'$ is trialkylsilyl so that the reaction for removing trialkylsilyl proceeds simultaneously, and, when required carrying out any of de-esterification, esterification subsequent to de-esterification, ester interchange reaction and salt-forming reaction.

11. A process for preparing a penicillin derivative as claimed in claim 10 comprising subjecting to reaction for removing trialkylsilyl a compound of the formula

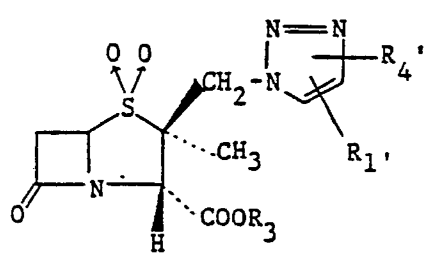

wherein $R_1'$ and $R_4'$ each represents hydrogen, tri $C_{1-6}$ alkylsilyl, carboxyl, a carboxyl group forming a pharmaceutically acceptable salt or an esterified carboxyl group, provided that at least one of $R_1'$ and $R_4$ is tri $C_{1-6}$ alkylsilyl, and when required, carrying out any of de-esterification, esterification subsequent to de-esterification, ester interchange reaction and salt-forming reaction.

12. A process for preparing a penicillin derivative represented by the following formula, and a pharmaceutically acceptable salt thereof and an ester thereof:

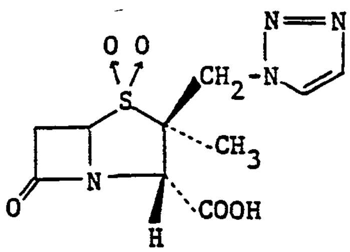

the process comprising reacting a compound of the formula

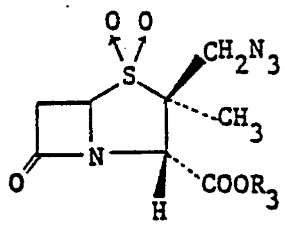

wherein $R_3$ represents a penicillin carboxyl-protecting group with a vinyl derivative of the formula

$$CH_2{=}CHR_6$$

wherein $R_6$ represents acyloxy so that reaction for removing acyloxy proceeds simultaneously, and, when required, carrying out any of de-esterification, esterification subsequent to de-esterification, ester interchange reaction and salt-forming reaction.

13. A β-lactamase inhibitor composition which contains as the active ingredient at least one of the penicillin derivative as defined in claim 1, and the pharmaceutically acceptable salt thereof and ester thereof.

**Patentansprüche**

1. Penicillinderivat der folgenden Formel und ein pharmazeutisch annehmbares Salz davon und ein Ester davon:

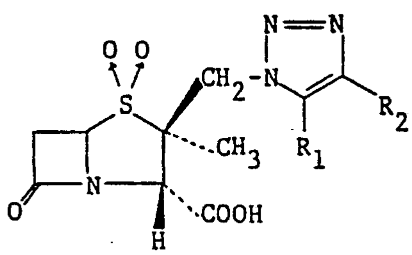

(I)

worin $R_1$ und $R_2$, die gleich oder verschieden sein können, Wasserstoffatome, Tri-$C_{1-6}$-alkylsilylgruppen, Carboxylgruppen oder Carboxylgruppen, die ein pharmazeutisch annehmbares Salz bilden, oder veresterte Carboxylgruppen bedeuten.

2. Derivat nach Anspruch 1, in dem einer der Reste $R_1$ und $R_2$ Wasserstoff und der andere eine veresterte Carboxylgruppe ist.

3. Derivat nach Anspruch 1, in dem $R_1$ und $R_2$ beide veresterte Carboxylgruppen sind.

4. Derivat nach Anspruch 1, in dem einer der Reste $R_1$ und $R_2$ Wasserstoff und der andere Wasserstoff, Carboxyl oder eine ein pharmazeutisch annehmbares Salz bildende Carboxylgruppe ist.

5. Derivat nach Anspruch 1, in dem irgendeiner der Reste $R_1$ und $R_2$ Tri-$C_{1-6}$-alkylsilyl ist.

6. Derivat nach irgendeinem der Ansprüche 1, 2 und 3, in dem die veresterte Carboxylgruppe Alkoxycarbonyl mit 1 bis 18 Kohlenstoffatomen in der Alkylgruppe ist.

7. Pharmazeutisch annehmbares Salz des Derivats nach Anspruchs 1, welches Salz ein Alkalimetallsalz, Erdalkalimetallsalz, organisches Aminsalz, basisches Aminosäuresalz oder Ammoniumsalz ist.

8. Pharmazeutisch annehmbarer Ester des Derivats nach Anspruch 1, welcher Ester in vivo leicht zur freien Säure hydrolysiert wird.

9. Verfahren zur Herstellung eines Penicillinderivats der folgenden Formel und eines pharmazeutisch annehmbaren Salzes davon und eines Esters davon:

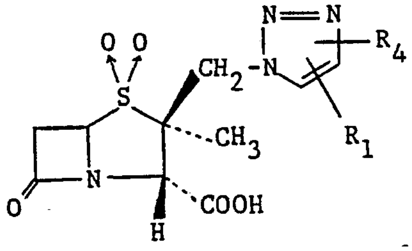

worin $R_1$ Wasserstoff, Tri-$C_{1-6}$-alkylsilyl, Carboxyl, ein pharmazeutisch annehmbares Salz bildendes Carboxyl oder verestertes Carboxyl bedeutet und $R_4$ eine Tri-$C_{1-6}$-alkylsilylgruppe, eine Carboxylgruppe, eine ein pharmazeutisch annehmbares Salz bildende Carboxylgruppe oder eine veresterte Carboxylgruppe bedeutet, welches Verfahren umfaßt die Umsetzung einer Verbindung der Formel

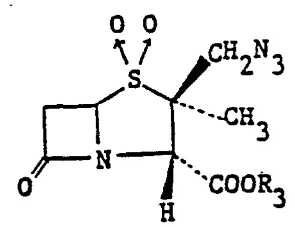

worin $R_3$ eine Penicillin-Carboxylschutzgruppe bedeutet, mit einem Acetylenderivat der Formel

$$R_1C{\equiv}CR_4$$

worin $R_1$ und $R_4$ wie oben definiert sind, und gegebenenfalls Durchführung einer Entesterung, Veresterung im Anschluß an die Entesterung, Umesterung oder Salzbildungsreaktion.

10. Verfahren zur Herstellung eines Penicillinderivats der folgenden Formel und eines pharmazeutisch annehmbaren Salzes davon und eines Esters davon:

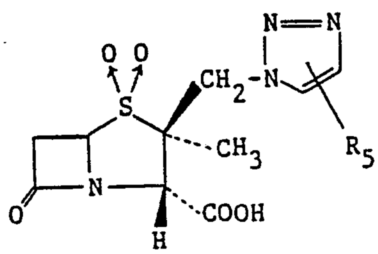

worin $R_5$ Wasserstoff, Carboxyl, ein pharmazeutisch annehmbares Salz bildendes Carboxyl oder verestertes Carboxyl bedeutet, welches Verfahren umfaßt die Umsetzung einer Verbindung der Formel

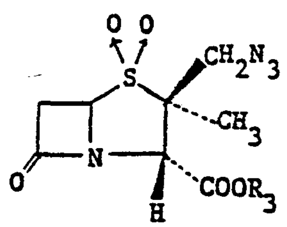

worin $R_3$ eine Penicillin-Carboxylschutzgruppe bedeutet, mit einem Acetylenderivat der Formel

$$R_1'C{\equiv}CR_4'$$

worin $R_1'$ und $R_4'$ jeweils Wasserstoff, Tri-$C_{1-6}$-alkylsilyl, Carboxyl, eine ein pharmazeutisch annehmbares Salz bildende Carboxylgruppe oder eine veresterte Carboxylgruppe bedeuten, mit der Maßgabe, daß mindestens einer der Reste $R_1'$ und $R_4'$ Trialkylsilyl ist, so daß die Reaktion zur Abspaltung von Trialkylsilyl gleichzeitig abläuft, und gegebenenfalls Durchführung einer Entesterung, Veresterung im Anschluß an die Entesterung, Umesterung oder Salzbildungsreaktion.

11. Verfahren zur Herstellung eines Penicillinderivats nach Anspruch 10, umfassend die Abspaltung von Trialkylsilyl aus einer Verbindung der Formel

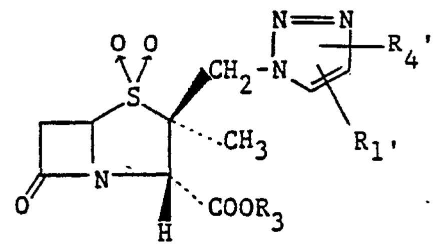

worin $R_1'$ und $R_4'$ jeweils Wasserstoff, Tri-$C_{1-6}$-alkylsilyl, Carboxyl, eine ein pharmazeutisch annehmbares Salz bildende Carboxylgruppe oder eine veresterte Carboxylgruppe bedeuten, mit der Maßgabe, daß mindestens einer der Reste $R_1'$ und $R_4'$ Tri-$C_{1-6}$-alkylsilyl ist, und gegebenenfalls Durchführung einer Entesterung, Veresterung im Anschluß an die Entesterung, Umesterung oder Salzbildungsreaktion.

12. Verfahren zur Herstellung eines Penicillinderivats der folgenden Formel und eines pharmazeutisch annehmbaren Salzes davon und eines Esters davon:

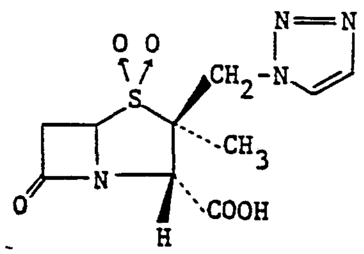

welches Verfahren umfaßt die Umsetzung einer Verbindung der Formel

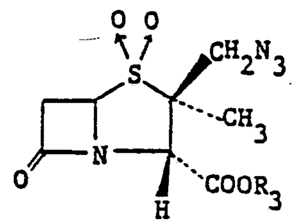

worin $R_3$ eine Penicillin-Carboxylschutzgruppe bedeutet, mit einem Vinylderivat der Formel

$$CH_2{=}CHR_6$$

worin $R_6$ Acyloxy bedeutet, so daß die Reaktion zum Entfernen von Acyloxy gleichzeitig abläuft, und gegebenenfalls Durchführung einer Entesterung, Veresterung im Anschluß an die Entesterung, Umesterung oder Salzbildungsreaktion.

13. β-Lactamaseinhibitor-Zusammensetzung, die als Wirkstoff mindestens eines der Penicillinderivate nach Anspruch 1, pharmazeutisch annehmbaren Salze und Ester davon enthält.

**Revendications**

1. Dérivés de pénicilline de formule I ci-dessous, ainsi que leurs sels convenant pour un usage pharmaceutique et leurs esters:

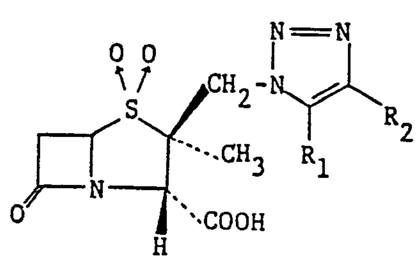

(I)

formule dans laquelle $R_1$ et $R_2$, qui peuvent être identiques ou différents l'un de l'autre, représentent des atomes d'hydrogène, des groupes tri-$C_{1-6}$-alkylsilyles, des groupes carboxyles ou des groupes carboxyles salifiés (sels acceptables en pharmacie) ou estérifiés.

2. Un dérivé selon la revendication 1 dans lequel parmi $R_1$ et $R_2$, l'un est l'hydrogène et l'autre un groupe ester carboxylique.

3. Un dérivé selon la revendication 1 dans lequel $R_1$ et $R_2$ sont tous deux des groupes carboxyliques estérifiés.

4. Un dérivé selon la revendication 1 dans lequel parmi $R_1$ et $R_2$ l'un est l'hydrogène et l'autre l'hydrogène, un groupe carboxyle ou un groupe carboxyle salifié.

5. Un dérivé selon la revendication 1 dans lequel, parmi $R_1$ et $R_2$, l'un quelconque est un groupe tri-$C_{1-6}$-alkylsilyle.

6. Un dérivé selon l'une quelconque des revendications 1 à 3 dans lequel le groupe carboxylique estérifié est un groupe alcoxycarbonyle dont l'alkyle a de 1 à 18 atomes de carbone.

7. Un sel pharmaceutiquement acceptable selon la revendication 1, qui est un sel de métal alcalin ou alcalino-terreux, d'une amine, d'un amino acide basique ou d'ammonium.

8. Un ester pharmaceutiquement acceptable d'un dérivé selon la revendication 1 qui s'hydrolyse facilement in-vivo en donnant l'acide libre.

9. Un procédé de préparation de dérivés de pénicilline de formule ci-dessous ainsi que de leurs sels pharmaceutiquement acceptables et d'esters de ces dérivés:

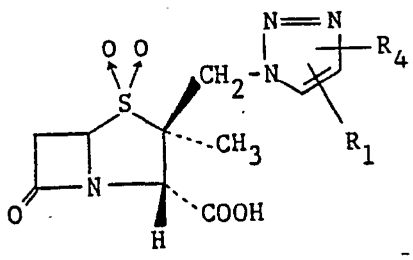

(formule dans laquelle $R_1$ représente l'hydrogène, un groupe tri-$C_{1-6}$-alkylsilyle, un groupe carboxyle ou un groupe carboxyle salifié ou estérifié, et $R_4$ un groupe tri-$C_{1-6}$-alkylsilyle, un groupe carboxyle ou un groupe carboxyle salifié ou estérifié), procédé dans lequel on fait réagir un composé de formule:

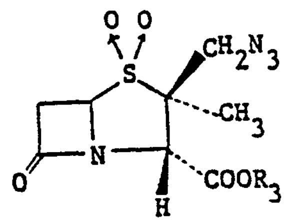

($R_3$ étant un groupe protégeant le carboxyle de pénicilline) avec un dérivé d'acétylène de formule

$$R_1C{\equiv}CR_4$$

($R_1$ et $R_4$ ayant les significations données ci-dessus) et le cas échéant on effectue une désestérification, une estérification après la désestérification, une transestérification ou une salification.

10. Un procédé de préparation d'un dérivé de pénicilline de formule ci-dessous, ainsi que de leurs sels pharmaceutiquement acceptables et d'esters de ces dérivés:

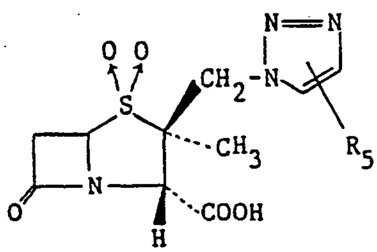

(formule dans laquelle $R_5$ représente l'hydrogène, un groupe carboxyle ou un groupe carboxyle salifié ou estérifié), procédé dans lequel on fait réagir un composé de formule:

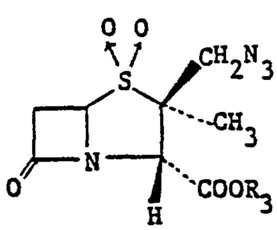

($R_3$ étant un groupe protégeant le carboxyle de pénicilline) avec un dérivé d'acétylène de formule

$$R_1'C{\equiv}CR_4'$$

($R_1'$ et $R_4'$ étant chacun l'hydrogène, un groupe tri-$C_{1-6}$-alkylsilyle, un groupe carboxyle ou un groupe carboxyle salifié ou estérifié, avec la condition que parmi $R_1'$ et $R_4'$ l'un au moins soit un groupe trialkylsilyle de manière que la réaction d'élimination du groupe trialkylsilyle se fasse en même temps), et le cas échéant on procède à une désestérification, à une estérification après la désestérification, à une transestérification ou à une salification.

11. Un procédé de préparation de dérivés de pénicilline selon la revendication 10, dans lequel on soumet à une réaction d'élimination du groupe trialkylsilyle un composé de formule:

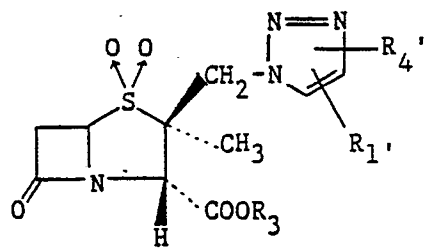

(dans laquelle $R_1'$ et $R_4'$ sont chacun l'hydrogène, un groupe tri-$C_{1-6}$-alkylsilyle, un groupe carboxyle ou un groupe carboxyle salifié ou estérifié, avec la condition que parmi $R_1'$ et $R_4'$ l'un au moins soit un groupe tri-$C_{1-6}$-alkylsilyle), et le cas échéant on procède à une désestérification, à une estérification après la désestérification, à une transestérification ou à une salification.

12. Un procédé de préparation d'un dérivé de pénicilline de formule ci-dessous, ainsi que de sels pharmaceutiquement acceptables et d'esters de ce dérivé:

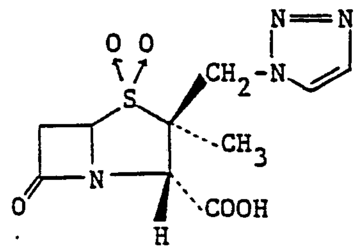

procédé dans lequel on fait réagir un composé de formule

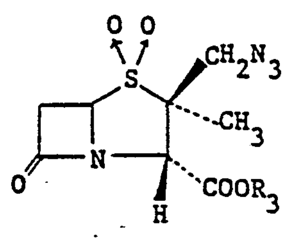

($R_3$ étant un groupe qui protège le carboxyle de pénicilline) avec un dérivé vinylique de formule

$$CH_2{=}CHR_6$$

$R_6$ étant un groupe acyloxy, de manière que la réaction d'élimination du groupe acyloxy se fasse en même temps, et le cas échéant on procède à une désestérification, à une estérification après la désestérification, à une transestérification ou à une salification.

13. Une composition qui inhibe l'action de la β-lactamase, composition comprenant comme ingrédient actif un ou plusieurs dérivés de pénicilline selon la revendication 1 ou sels pharmaceutiquement acceptables ou esters de ces dérivés.